# FASCICULE DE BREVET EUROPEEN

(11) **EP 2 967 034 B1**
(45) Date de publication et mention de la délivrance du brevet: **22.02.2017**
(21) Numéro de dépôt: 14715362.1
(22) Date de dépôt: 12.03.2014
(51) Int. Cl.: A01N 25/30, A01N 25/04, A01N 47/06, A01N 43/28, A01N 43/32, A01P 21/00

(54) **UTILISATIONS D'ESTERS CARBONIQUES DE GLYCÉROL ACYLÉS EN AGRICULTURE**
VERWENDUNG VON ACYLIERTEN CARBONSÄUREESTERN VON GLYCEROL IN DER LANDWIRTSCHAFT
USE OF ACYLATED CARBONIC ESTERS OF GLYCEROL IN AGRICULTURE

(30) Priorité: 14.03.2013 FR 1352301
(43) Date de publication de la demande: 20.01.2016
(73) Titulaire: Agronutrition, 31390 Carbonne (FR); Institut National Polytechnique De Toulouse (INPT), 31400 Toulouse (FR); Institut National de la Recherche Agronomique (INRA), 75007 Paris (FR)
(72) Inventeur: ATTIA, Faouzi, F-31450 Ayguesvives (FR); CABANES, Cédric, F-31320 Pechabou (FR); GUENIER, Monique, F-31650 Saint Orens (FR); MOULOUNGUI, Zéphirin, F-31000 Toulouse (FR); ZEBIB, Bachar, F-31280 Aigrefeuille (FR); VALENTIN, Romain, F-31400 Toulouse (FR); ABDEL BAKI, Zaher, F-31500 Toulouse (FR)
(74) Mandataire: Cabinet BARRE LAFORGUE & associés
(86) Numéro de dépôt international: PCT/FR2014/050567
(87) Numéro de publication internationale: WO 2014/140484

(56) Documents cités:
- EP-A1- 1 366 663
- WO-A1-00/51427
- FR-A1- 2 874 217
- FR-A1- 2 880 025
- DATABASE CAPLUS [Online] CHEMICAL ABSTRACTS SERVICE, COLUMBUS, OHIO, US; SUZUKI, TADAYUKI ET AL: "Fertilizers for improving carbohydrate content in crops", XP002699043, extrait de STN Database accession no. 2002:708748 & JP 2006 182684 A (KAO CORP., JAPAN) 13 juillet 2006 (2006-07-13)

## Description

L'invention concerne de nouvelles utilisations d'esters carboniques de glycérol.

Des esters carboniques de glycérol sont connus pour pouvoir être utilisés comme additifs dans des lubrifiants industriels -notamment des lubrifiants pour automobiles- et comme additifs dans les boues de forage pétrolier.

En particulier, on connaît de Mouloungui et al. (2001), Eur. J. Lipid Sci. Technol. , 103, 216-222 "Study of the acyl transfer reaction : Structure and properties of glycerol esters" les propriétés de stabilité à la chaleur et de résistance à l'oxydation de l'acétate de carbonate cyclique de glycérol, du butyrate de carbonate cyclique de glycérol, de l'octanoate de carbonate cyclique de glycérol, du dodécanoate de carbonate cyclique de glycérol, du palmitate de carbonate cyclique de glycérol, du stéarate de carbonate cyclique de glycérol et de l'oléate de carbonate cyclique de glycérol et leurs applications à titre de lubrifiants et/ou d'additifs d'une composition lubrifiante.

On connait de JP20066182684, de EP1366663 et de WO 00/51427 des utilisations de carbonate cyclique de glycérol en agriculture.

L'invention telle que définie dans les revendications vise à proposer de nouvelles utilisations d'esters carboniques cycliques de glycérol α/α'-acylés et de façon plus générale à proposer de nouvelles utilisations d'esters carboniques de glycérol.

Pour ce faire, l'invention concerne un procédé de traitement de végétaux comprenant l'application d'au moins un composé, dit ester carbonique de glycérol, comprenant au moins une fonction ester carboxylique formée par un groupement dérivé de l'acide carbonique et au moins un groupement dérivé du glycérol, à l'exception du carbonate cyclique de glycérol à cinq chaînons , tel que défini dans les revendications.

L'invention concerne un procédé de traitement de végétaux comprenant l'application d'au moins un tel ester carbonique de glycérol sur un végétal.

Par carbonate cyclique de glycérol à cinq chaînons, on entend l'ester carbonique de glycérol de formule (VI) suivante :

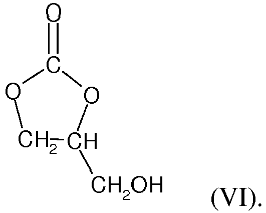

L'invention concerne donc l'utilisation comme intrant en agriculture d'au moins un composé, dit ester carbonique de glycérol, comprenant au moins une fonction ester carboxylique formée par un groupement dérivé de l'acide carbonique et au moins un groupement dérivé du glycérol, à l'exception du carbonate cyclique de glycérol à cinq chaînons, tel que défini dans les revendications. Plus particulièrement, l'invention concerne l'utilisation, à titre d'intrant agricole, de tels ester(s) carbonique(s) de glycérol appliqué(s) à des végétaux.

Dans tout le texte, on désigne par :
- groupement "dérivé de glycérol" tout groupement de formule suivante : 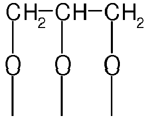 dans laquelle au moins un atome d'hydrogène des groupements hydroxyles du glycérol est substitué par un groupement organique distinct de l'hydrogène, et par ;
- groupement "dérivé de l'acide carbonique", tout groupement de formule suivante : 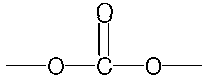 dans laquelle au moins un atome d'hydrogène de l'acide carbonique est substitué par un groupement organique distinct de l'hydrogène.

Avantageusement et selon l'invention, au moins un ester carbonique de glycérol est choisi dans le groupe formé des esters carboniques linéaires de glycérol et des esters carboniques cycliques de glycérol.

Un ester carbonique de glycérol peut être choisi dans le groupe formé des esters carboniques linéaires de glycérol présentant au moins un groupement d'atomes de formule (I) générale suivante : 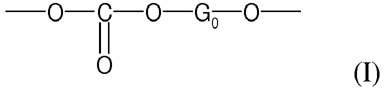 dans laquelle G₀ est choisi dans le groupe formé :
▪ des groupements propylènes α/α'-acylés de formule (II) générale suivante : 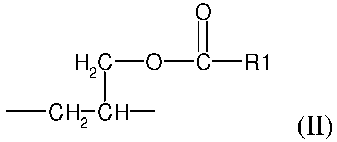
▪ des groupements propylènes β-acylés de formule (III) générale suivante : 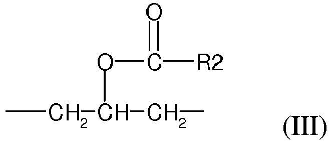
▪ du groupement propylène α/α'-hydroxylé de formule (IV) suivante : 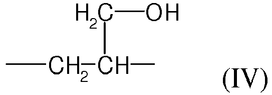
▪ du groupement propylène β-hydroxylé de formule (V) suivante : 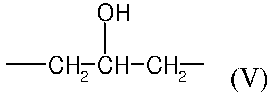 R1 et R2 étant des groupements organiques formés d'éléments choisis dans le groupe formé du carbone (C), de l'hydrogène (H) et de l'oxygène (O).

Un ester carbonique de glycérol peut être choisi dans le groupe formé des esters carboniques linéaires de glycérol présentant au moins un groupement d'atomes de formule (I') générale suivante : 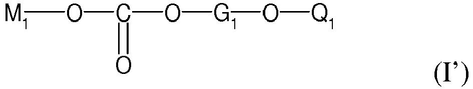 dans laquelle :
- G₁ est choisi dans le groupe formé :
   ▪ des groupements propylènes α/α'-acylés de formule (II) générale ;
   ▪ des groupements propylènes β-acylés de formule (III) générale ;
   ▪ du groupement propylène α/α'-hydroxylé de formule (IV) ;
   ▪ du groupement propylène β-hydroxylé de formule (V) ;
- Q₁ est choisi dans le groupe formé de l'hydrogène (H) et des groupements organiques formés d'au moins deux atomes liés par des liaisons covalentes, lesdits atomes appartenant au groupe formé du carbone (C), de l'hydrogène (H) et de l'oxygène (O), et ;
- M₁ représente un groupement organique formé d'au moins deux atomes liés par des liaisons covalentes, lesdits atomes appartenant au groupe formé du carbone (C), de l'hydrogène (H) et de l'oxygène (O).

Un ester carbonique de glycérol peut être un ester carbonique cyclique de glycérol α/α'-acylé de formule (VIII) générale suivante : 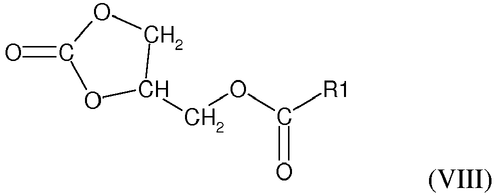 dans laquelle R1 est un groupement organique formé d'éléments choisis dans le groupe formé du carbone (C), de l'hydrogène (H) et de l'oxygène (O).

Un ester carbonique de glycérol peut être un ester carbonique cyclique de glycérol β-acylé de formule (IX) générale suivante : 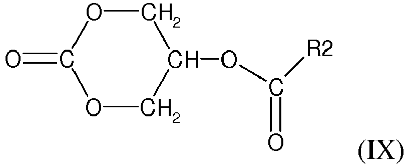 dans laquelle R2 est un groupement organique formé d'éléments choisis dans le groupe formé du carbone (C), de l'hydrogène (H) et de l'oxygène (O). Un ester carbonique de glycérol peut être un ester carbonique cyclique de glycérol à six chaînons β-acylé.

Un ester carbonique de glycérol peut être choisi dans le groupe formé des esters carboniques linéaires de glycérol acylés -notamment des oligomères carboniques linéaires de glycérol- de formule (X) générale suivante : 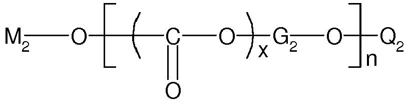 (X) dans laquelle ;
- x est un nombre entier égal à 0 ou à 1 pouvant varier dans la formule (X) selon chaque groupement de formule (X-a) : 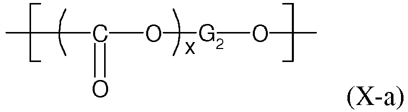 x n'étant pas toujours nul ;
- n est un nombre entier compris entre 1 et 20 -notamment compris entre 1 et 10-, bornes incluses ;
- Q₂ est choisi dans le groupe formé de l'hydrogène (H) et des groupements organiques formés d'au moins deux atomes liés par des liaisons covalentes, lesdits atomes appartenant au groupe formé du carbone (C), de l'hydrogène (H) et de l'oxygène (O), et ;
- M₂ représente un groupement organique formé d'au moins deux atomes liés par des liaisons covalentes, lesdits atomes appartenant au groupe formé du carbone (C), de l'hydrogène (H) et de l'oxygène (O), et ;
- G₂ représente un groupement d'atomes choisi dans le groupe formé :
   ▪ des groupements propyle α/α'-acylés de formule (II) générale, et ;
   ▪ des groupements propyle β-acylés de formule (III) générale.

L'ester carbonique de glycérol de formules (I), (I') ou (X) présente une masse molaire supérieure à 400 g/mole. Avantageusement, l'ester carbonique linéaire de glycérol α/α'-acylé de formule générale (X) présente une masse molaire supérieure à 400 g/mole.

Les groupements R1 et R2 d'esters carboniques de glycérol peuvent être des groupements hydrocarbonés aliphatiques comprenant de 1 à 25 atomes de carbone. Avantageusement, R1 et R2 sont choisis indépendamment l'un de l'autre. R1 et R2 peuvent donc être identiques ou différents.

En particulier, les groupements R1 et R2 peuvent être choisis dans le groupe formé du méthyle (-CH₃), de l'éthyle (-CH₂-CH₃), du *n*-propyle (-CH₂-CH₂-CH₃), de l'*iso*-propyle (-CH(CH₃)₂), du *n*-butyle (-CH₂-CH₂-CH₂-CH₃), de l'*iso*-butyle (-CH₂-CH(CH₃)₂), du *tertio*-butyle (-C(CH₃)₃), du *n*-pentyle (-(CH₂)₄-CH₃), du *n*-hexyle (-(CH₂)₅-CH₃), du *n*-heptyle (-(CH₂)₆-CH₃), du *n*-octyle (-(CH₂)₇-CH₃), du *n*-nonyle (-(CH₂)₈-CH₃), du *n*-décyle (-(CH₂)₉-CH₃), du *n*-undécyle (-(CH₂)₁₀-CH₃), du *n*-dodécyle (-(CH₂)₁₁-CH₃), du *n*-tridécyle (-(CH₂)₁₂-CH₃), du *n*-tétradécyle (-(CH₂)₁₃-CH₃), du *n*-pentadécyle (-(CH₂)₁₄-CH₃), du *n*-hexadécyle (-(CH₂)₁₅-CH₃), du *n*-heptadécyle (-(CH₂)₁₆-CH₃), du *n*-octadécyle (-(CH₂)₁₇-CH₃), du *n*-nonadécyle (-(CH₂)₁₈-CH₃), du *n*-dodécadécyle (-(CH₂)₁₉-CH₃).

Les groupements R1 et R2 peuvent aussi être choisis dans le groupe formé des alkyles insaturés -notamment d'un groupement 9-ène-decyle (-CH=CH-(CH₂)₇-CH₃) et d'un groupement 9-ène-heptadecyle (-(CH₂)₇-CH=CH-(CH₂)₇-CH₃).

De tels esters carboniques cycliques de glycérol α/α'-acylés ou β-acylés sont des composés multifonctionnels en cela qu'ils sont à la fois des composés solvants et des composés.

L'invention vise donc un procédé de traitement de végétaux comprenant l'application d'une composition de traitement comprenant au moins un ester carbonique de glycérol -notamment au moins un ester carbonate de glycérol selon l'une au moins des formules (I), (I'), (VIII), (IX) ou (X)- en contact avec lesdits végétaux.

Les inventeurs ont constaté que les esters carboniques de glycérol lorsqu'ils sont mis en contact avec des végétaux -notamment mais non exclusivement avec les parties aériennes de végétaux- d'une part ne nuisent pas à la croissance et au développement des végétaux, et présentent même, d'autre part, de nombreux avantages en tant qu'intrants agricoles. En particulier ils ne détériorent pas la cuticule des feuilles des végétaux, n'entrainent pas de brûlure des végétaux lorsqu'ils sont appliqués sur les parties aériennes de végétaux.

Les esters carboniques de glycérol sont adaptés pour pouvoir être appliqués sur des végétaux et satisfont les normes de protection et de préservation de l'environnement. Ils ne présentent pas en eux-mêmes de toxicité pour la santé humaine ou animale. Ils sont biodégradables, ils ne présentent pas de risque de s'accumuler dans les sols et de contaminer ou de détériorer l'environnement lors de leur application sur des végétaux et/ou lors de leur introduction dans le sol -par exemple, par lavage par les eaux de pluie ou par arrosage- et par ruissellement. Les esters carboniques de glycérol peuvent être "biosourcés", c'est-à-dire obtenus à partir de ressources naturelles -notamment de ressources végétales- peu onéreuses et renouvelables. Ils présentent donc un faible coût de revient et peuvent être appliqués de façon économique en plein champ sur des végétaux. En outre, ils présentent des propriétés d'adjuvant et d'amélioration de l'efficacité de substances actives. À ce titre, ils peuvent permettre de réduire les doses de ces substances actives à appliquer sur les cultures de végétaux et dans l'environnement et contribuent à la préservation de cet environnement.

Dans un mode de réalisation d'un procédé de traitement de végétaux selon l'invention on applique à des végétaux une composition de traitement comprenant au moins un ester carbonique de glycérol, tel que défini dans les revendications et optionnellement un excipient "phyto-acceptable", c'est-à-dire compatible avec son application aux végétaux et acceptable par ces derniers. Avantageusement et selon l'invention on applique au moins cet ester carbonique de glycérol -notamment une telle composition de traitement- au contact de végétaux à tout stade de leur développement ou de leur croissance. On peut appliquer au moins cet ester carbonique de glycérol -notamment une telle composition de traitement- sur des végétaux au stade de semence, ou sur des végétaux au stade de plantules, c'est-à-dire sur une jeune plante ne comportant qu'un nombre limité de feuilles, ou sur des végétaux juvéniles -notamment sur des végétaux à un stade antérieur à la floraison-, ou sur des végétaux en cours de floraison (avant, pendant ou après pollinisation), ou sur des végétaux après fécondation, ou sur des végétaux en cours de fructification. On peut également appliquer au moins cet ester carbonique de glycérol -notamment une telle composition de traitement- dans le substrat de culture de végétaux -par exemple dans le sol- de façon à permettre le contact d'au moins un ester carbonique de glycérol -notamment d'une telle composition de traitement-avec les parties souterraines -notamment par exemple les racines, les rhizomes, les tubercules- des végétaux. On peut également appliquer au moins cet ester carbonique de glycérol -notamment une telle composition de traitement- dans un milieu nutritif adapté pour pouvoir être apporté au substrat de culture de végétaux.

Avantageusement et selon l'invention, on applique au moins cet ester carbonique de glycérol au contact de parties souterraines de végétaux.

Avantageusement et selon l'invention, on applique au moins cet ester carbonique de glycérol -notamment une telle composition de traitement- au contact de parties aériennes de végétaux en culture. En particulier, on applique au moins cet ester carbonique de glycérol -notamment une telle composition de traitement- sur les fleurs, sur les fruits, sur les feuilles, sur les tiges de végétaux en culture.

Il est en particulier possible d'appliquer au moins cet ester carbonique de glycérol -notamment une telle composition de traitement- sur des végétaux en culture en plein champ ou sur des végétaux en culture sous serre ou sur des végétaux en culture hors sol.

Avantageusement et selon l'invention, on applique au moins cet ester carbonique de glycérol -notamment une telle composition de traitement- sur une culture maraîchère, sur une culture industrielle ou sur une grande culture.

Avantageusement et selon l'invention, on applique au moins cet ester carbonique de glycérol -notamment une telle composition de traitement- au contact d'un végétal choisi dans le groupe formé des arbres fruitiers -notamment des oliviers, des abricotiers, des cerisiers, des cognassiers, des amandiers, des figuiers, des noisetiers, des noyers, des pêchers, des poiriers, des pommiers, des pruniers, de la vigne et des agrumes-, des arbres et arbustes d'ornementation, des plantes potagères -notamment des asperges, des aubergines, des blettes, des betteraves potagères, des carottes, du céleri, de la chicorée, des endives, des crucifères ou brassicacées (par exemple les choux), des concombres, des cornichons, des courgettes, des échalotes, des oignons, des fèveroles, des féveroles de printemps, des féveroles d'hiver, des fraisiers, des framboisiers, des haricots, des laitues, des chicorées frisées, des chicorées scaroles, du houblon, des lentilles, de la luzerne, de la mâche, du maïs, des melons, des navets, des poireaux, des pois, des poivrons, des pommes de terre, des radis, des rutabagas, du soja, du tabac, des tomates, des tournesols-, des céréales -notamment du blé, du colza, du lin, du lin oléagineux, du lin textile, de l'orge, du sorgho-, des cultures florales diverses -notamment des chrysanthèmes, des hortensias, des oeillets, des rosiers, des tulipes-et des plantes aromatiques -notamment du persil, de l'ail, de la ciboulette-.

Ces esters carboniques de glycérol peuvent en particulier contribuer à améliorer de façon surprenante l'efficacité d'une composition de traitement de végétaux. Avantageusement et selon l'invention, on utilise ainsi au moins un ester carbonique de glycérol à titre d'adjuvant de vectorisation d'au moins un agent actif de traitement des végétaux autre qu'un ester carbonique de glycérol, notamment un agent actif choisi dans le groupe formé des agents actifs sur la germination de semences, des agents de contrôle de la croissance, des agents de développement de végétaux, des agents de stimulation de la photosynthèse et des nutriments pour végétaux.

Avantageusement et selon l'invention, on applique au moins cet ester carbonique de glycérol en association avec au moins un agent actif autre qu'un ester carbonique de glycérol, choisi dans le groupe formé des agents actifs sur la germination de semences, des agents de contrôle de la croissance, des agents de développement de végétaux, des agents de stimulation de la photosynthèse et des nutriments pour végétaux.

Les esters carboniques de glycérol peuvent en particulier contribuer à améliorer de façon surprenante l'efficacité d'une composition nutritive de végétaux. Avantageusement et selon l'invention, on utilise ainsi au moins cet ester carbonique de glycérol à titre d'adjuvant de vectorisation d'au moins un élément nutritif pour des végétaux. L'invention concerne à ce titre en particulier un procédé de traitement de végétaux comprenant l'application d'au moins cet ester carbonique de glycérol en association avec au moins un agent nutritif des végétaux.

L'application d'au moins cet ester carbonique de glycérol peut être effectuée séparément de l'application d'au moins un tel autre agent actif (notamment nutritif) -en particulier avec des compositions de traitement distinctes, appliquées simultanément ou au contraire successivement-. Avantageusement et selon l'invention l'application d'au moins cet ester carbonique de glycérol peut être effectuée simultanément à l'application d'au moins un tel autre agent actif (notamment nutritif) -en particulier par application d'une même composition de traitement, notamment nutritive-. Avantageusement et selon l'invention, on utilise au moins cet ester carbonique de glycérol dans une composition de traitement, notamment nutritive, destinée à être appliquée à des végétaux et comprenant en outre au moins un tel autre agent de traitement, notamment nutritif, des végétaux. Dans une composition de traitement, notamment nutritive, selon l'invention, au moins cet ester carbonique de glycérol peut faire office d'excipient phyto-acceptable. En variante selon l'invention, ladite composition de traitement, notamment nutritive, comprend en outre un excipient phyto-acceptable autre qu'un ester carbonique de glycérol.

### 1. Traitement de semences

Un premier aspect de l'invention concerne l'utilisation d'au moins un ester carbonique de glycérol selon les revendications -notamment d'une composition de traitement comprenant au moins un ester carbonique de glycérol- pour le traitement de semences d'au moins un végétal. L'invention concerne donc un procédé de traitement des semences d'au moins un végétal dans lequel on met en contact les semences avec au moins un ester carbonique glycérol selon les revendications -notamment avec une composition de traitement de semences comprenant au moins un ester carbonique de glycérol selon les revendications-.

Avantageusement et selon l'invention, on applique au moins cet ester carbonique de glycérol -notamment au moins un ester carbonique de glycérol de formule (I), (I'), (VIII), (IX) ou (X)- au contact de semences de végétaux. On applique sur des semences de végétaux une composition de traitement de semences comprenant au moins cet ester carbonique de glycérol.

Un procédé de traitement de semences selon l'invention peut être adapté pour pouvoir préserver les semences de la germination préalablement à leur enfouissement dans le sol et/ou pour activer la germination des semences après leur enfouissement dans le sol.

Avantageusement, dans une première variante de ce premier aspect de l'invention, on réalise un enrobage des semences avec une composition de traitement de semences comprenant au moins cet ester carbonique de glycérol et au moins un micro-organisme choisi dans le groupe formé des champignons mycorhiziens, des rhizobactéries (par exemple *Azotobacter*, *Azospirillum, Pseudomonas*). En particulier, l'ester carbonique de glycérol permet l'adhésion des micro-organismes sur les semences et le développement des micro-organismes lors de la germination des semences.

En particulier, un tel micro-organisme peut être un micro-organisme connu sous le nom de PGPR ("*Plant Growth Promoting Rhizobacteria*") et comprenant des bactéries de la rhizosphère qui sont bénéfiques à la croissance et la santé des végétaux.

Une composition de traitement de semences selon l'invention peut comprendre une proportion massique d'au moins cet ester carbonique de glycérol inférieure à 50 %, en particulier inférieure à 10 %, de préférence comprise entre 0,1 % et 5 %.

Dans une deuxième variante de ce premier aspect de l'invention, on applique une composition de traitement de semences -notamment une composition de traitement de semences comprenant au moins cet ester carbonique de glycérol et au moins un micro-organisme choisi dans le groupe formé des champignons mycorhiziens et des rhizobactéries- sur les semences lors de leur semis, c'est-à-dire simultanément au semis, ou postérieurement au semis.

Selon une troisième variante, on applique sur des semences d'au moins un végétal, une composition de traitement comprenant au moins cet ester carbonique de glycérol et au moins un agent de stimulation de la germination de ces semences autre qu'un ester carbonique de glycérol.

L'invention vise aussi une composition de traitement de semences de végétaux comprenant au moins un ester carbonique de glycérol selon les revendications -notamment au moins un ester carbonique de glycérol de formule (I), (I'), (VIII), (IX) ou (X)- et au moins une substance active sur la germination de semences - notamment au moins un micro-organisme- autre qu'un ester carbonique de glycérol. Une composition de traitement de semences de végétaux selon l'invention peut aussi comprendre en outre un excipient phyto-acceptable autre qu'un ester carbonique de glycérol.

Un procédé de traitement des semences de végétaux et une composition de traitement de végétaux selon l'invention permettent notamment de :
- stimuler la germination des semences et la croissance des végétaux issus de ces semences,
- augmenter la résistance des végétaux issus de ces semences vis-à-vis d'organismes phyto-pathogènes,
- améliorer l'enracinement des végétaux,
- favoriser l'assimilation et la fixation d'éléments nutritifs, notamment choisis parmi l'azote (N), le phosphore (P), le potassium (K) et le fer (Fe) et les oligoéléments,
- favoriser la fixation de l'azote atmosphérique,
- favoriser la formation d'une symbiose entre ledit micro-organisme et les végétaux issus de ces semences, et ;
- de façon générale, augmenter le rendement de production des végétaux.

L'invention vise aussi une composition de traitement de semences de végétaux comprenant au moins un ester carbonique de glycérol selon les revendications et, le cas échéant, au moins un micro-organisme choisi dans le groupe formé des champignons mycorhiziens et des rhizobactéries et/ou au moins une substance active -notamment stimulante- sur la germination de semences autre(s) qu'un ester carbonique de glycérol. Une composition de traitement de semence de végétaux peut aussi comprendre optionnellement un excipient phyto-acceptable autre qu'un ester carbonique de glycérol.

### 2. Traitement de stimulation de la photosynthèse

Un autre aspect de l'invention concerne l'utilisation d'au moins un ester carbonique de glycérol selon les revendications -notamment au moins un ester carbonique de glycérol de formule (I), (I'), (VIII), (IX) ou (X)- pour la stimulation de la photosynthèse de végétaux. L'invention concerne donc un procédé de traitement pour la stimulation de la photosynthèse de végétaux comprenant l'application d'au moins cet ester carbonique de glycérol -notamment d'une composition de stimulation de la photosynthèse comprenant au moins cet ester carbonique de glycérol-. Cet aspect de l'invention vise aussi l'utilisation d'une composition de stimulation de la photosynthèse comprenant au moins un ester carbonate de glycérol -notamment au moins un ester carbonique de glycérol de formule (I), (I'), (VIII), (IX) ou (X)- et au moins un agent actif sur la stimulation de la photosynthèse autre qu'un ester carbonique de glycérol. L'invention concerne donc un procédé de stimulation de la photosynthèse de végétaux dans lequel on applique au moins cet ester carbonique de glycérol -notamment une composition de stimulation de la photosynthèse comprenant un tel ester carbonique de glycérol-. Cet aspect de l'invention concerne donc aussi une composition de stimulation de la photosynthèse comprenant au moins cet ester carbonique de glycérol -notamment au moins un ester carbonique de glycérol de formule (I), (I'), (VIII), (IX) ou (X)-, et au moins un agent actif sur la stimulation de la photosynthèse autre qu'un ester carbonique de glycérol, et, optionnellement, un excipient phyto-acceptable autre qu'un ester carbonique de glycérol.

### 3. Traitement nutritif / fertilisation

Un autre aspect de l'invention concerne l'utilisation d'au moins un ester carbonique de selon les revendications -notamment au moins un ester carbonique de glycérol de formule (I), (I'), (VIII), (IX) ou (X)- dans un traitement nutritif, en particulier pour le contrôle de la croissance et/ou du développement de végétaux, notamment par fertilisation et/ou par application sur les végétaux.

L'invention concerne ainsi un procédé de traitement nutritif de végétaux. L'invention concerne en particulier un procédé de traitement nutritif de végétaux comprenant l'application d'au moins cet ester carbonique de glycérol -notamment d'une composition nutritive-. Selon l'invention, on applique une composition nutritive sur des végétaux dans l'un quelconque de leurs stades de croissance et/ou de développement.

Avantageusement et selon l'invention, on applique au contact de végétaux une composition nutritive comprenant au moins cet ester carbonique de glycérol et au moins un solide à l'état divisé comprenant au moins un composé choisi dans le groupe formé des éléments nutritifs des végétaux.

Avantageusement, on applique une composition nutritive comprenant au moins cet ester carbonique de glycérol et au moins un agent de traitement nutritif de végétaux autre qu'un ester carbonique de glycérol.

L'invention s'étend à une composition nutritive comprenant au moins un ester carbonique de glycérol selon les revendications -notamment au moins un ester carbonique de glycérol de formule (I), (I'), (VIII), (IX) ou (X)- et au moins un agent nutritif de végétaux autre qu'un ester carbonique de glycérol et, optionnellement, un excipient phyto-acceptable autre qu'un ester carbonique de glycérol. Une composition nutritive selon l'invention peut se présenter sous toute forme appropriée à son administration au contact des végétaux et/ou de leur substrat, notamment sous forme de granulés, de poudre, de solution ou suspension liquide à épandre ou pulvériser, ou autre.

L'invention concerne en particulier l'utilisation d'au moins cet ester carbonique de glycérol -notamment d'une composition nutritive comprenant au moins cet ester carbonique de glycérol- pour le traitement nutritif de végétaux par application directe sur des parties aériennes et/ou sur des parties souterraines des végétaux. Avantageusement, on applique une composition nutritive comprenant au moins cet ester carbonique de glycérol et au moins un élément nutritif des végétaux autre qu'un ester carbonique de glycérol. La composition nutritive selon l'invention est avantageusement choisie pour pouvoir permettre la libération d'au moins un élément nutritif à travers la cuticule de végétaux. Avantageusement et selon l'invention, on applique la composition nutritive sur au moins une partie aérienne des végétaux -notamment sur au moins une partie du feuillage des végétaux-. Une composition nutritive selon l'invention est en particulier adaptée pour pouvoir être appliquée sur les parties aériennes de végétaux. Par exemple, on applique une composition nutritive liquide par nébulisation (au pulvérisateur). En particulier, on peut appliquer la composition nutritive selon l'invention notamment sur des plantes potagères ou sur les plantes ornementales en culture. On peut ainsi utiliser la composition nutritive selon l'invention notamment en horticulture.

Les inventeurs ont observé qu'une composition nutritive comprenant au moins un ester carbonique de glycérol améliore de façon surprenante l'assimilation d'éléments nutritifs par la plante. Sans être liée par cette explication possible, cette assimilation pourrait provenir du fait qu'un ester carbonique de glycérol permet de contrôler les interactions de gouttelettes de la composition nutritive formées en surface de parties aériennes -notamment en surface des feuilles- de végétaux. Une telle composition nutritive paraît en effet diminuer la valeur de l'angle de goutte formée en surface de feuilles de vigne, en surface de feuilles de soja et en surface de feuilles de colza, en comparaison avec une composition nutritive exempte d'ester carbonique de glycérol.

Avantageusement et selon une variante, l'invention concerne un procédé de fertilisation dans lequel on applique au moins un ester carbonique de glycérol selon les revendications à titre d'amendement du substrat de culture -par exemple du sol. Avantageusement et selon l'invention, on applique une composition nutritive de fertilisation choisie pour favoriser l'assimilation des éléments minéraux par les racines, et améliorer la croissance et le développement racinaire des végétaux. Il est aussi possible par exemple d'utiliser une telle composition nutritive de fertilisation sur des végétaux lors de leur bouturage ou encore lors de leur rempotage des végétaux.

L'invention s'étend en particulier à une composition nutritive de fertilisation comprenant au moins cet ester carbonique de glycérol -notamment au moins un ester carbonique de glycérol de formule (I), (I'), (VIII), (IX) ou (X)- et au moins un micro-organisme choisi dans le groupe formé des champignons mycorhiziens et des rhizobactéries. Une composition nutritive de fertilisation selon l'invention peut aussi comprendre optionnellement un excipient phyto-acceptable autre qu'un ester carbonique de glycérol.

Un tel traitement nutritif (par application directe et/ou fertilisation) de végétaux permet d'apporter auxdits végétaux les éléments nutritifs nécessaires à leur développement et à leur croissance en vue d'obtenir un rendement optimal de production -compte tenu de la qualité du sol, du climat, du potentiel génétique desdits végétaux et des apports hydriques- et des qualités nutritionnelles et organoleptiques optimales.

Avantageusement on applique une composition nutritive comprenant au moins cet ester carbonique de glycérol et au moins un élément nutritif -notamment au moins un élément nutritif minéral utile au développement et/ou à la croissance de ces végétaux-.

Avantageusement, on applique une composition nutritive selon l'invention comprenant au moins cet ester carbonique de glycérol selon un dosage choisi pour satisfaire la ration nutritionnelle des végétaux. En particulier, on applique une composition nutritive selon l'invention sur des végétaux de façon à pallier une carence nutritionnelle desdits végétaux en macroélément (par exemple en azote, en phosphore, en potassium), en méso-élément (par exemple en calcium, en soufre, en magnésium) ou en oligoélément (par exemple en manganèse, en cuivre, en fer, en bore, en molybdène, en zinc, en nickel, en sélénium, en silicium ou en sodium).

Avantageusement, il est possible d'appliquer une composition nutritive selon l'invention à une plante présentant une carence en au moins un élément nutritif en vue de pallier à cette carence. Dans ce cas, on applique avantageusement au moins un ester carbonique de glycérol en association avec au moins un agent nutritif choisi pour pallier ladite carence.

Il est possible d'appliquer la composition nutritive selon l'invention sous toute forme possible, par exemple sous forme d'une poudre, sous forme de granulés -notamment sous forme de micro-granulés-, sous forme d'une solution liquide ou sous forme d'une suspension concentrée dudit agent nutritif, ou autre.

Dans un mode de réalisation de l'invention, on prépare tout d'abord une suspension colloïdale concentrée d'au moins un solide à l'état divisé comprenant au moins un élément nutritif des végétaux dans une phase liquide comprenant au moins un ester carbonique de glycérol, selon les revendications puis on dilue ladite suspension colloïdale concentrée dans l'eau de façon à former une composition nutritive à appliquer pour le traitement de végétaux.

Avantageusement, on prépare une suspension colloïdale concentrée comprenant au moins cet ester carbonique de glycérol, du glycérol, de l'eau et un solide à l'état divisé comprenant au moins un élément nutritif des végétaux choisi dans le groupe formé de l'azote (N), du phosphore (P), du potassium (K), du calcium (Ca), du magnésium (Mg), su soufre (S), du fer (Fe), du cuivre (Cu), du sodium (Na), du zinc (Zn), du bore (B), du molybdène (Mo), du silicium (Si), du sélénium (Se) et du nickel (Ni), par exemple du carbonate de manganèse (MnCO₃) et/ou du sulfate de manganèse (MnSO₄) et on dilue ladite suspension colloïdale concentrée dans une phase aqueuse de façon à former la composition nutritive. À titre d'exemple, on réalise une dilution de 1 à 2 volume(s) de suspension colloïdale concentrée dans 100 volumes d'eau pour former la composition nutritive.

L'invention concerne aussi une suspension colloïdale concentrée et une composition nutritive comprenant au moins cet ester carbonique de glycérol et au moins un solide à l'état divisé comprenant au moins un élément nutritif des végétaux choisi dans le groupe formé de l'azote (N), du phosphore (P), du potassium (K), du calcium (Ca), du magnésium (Mg), du soufre (S), du fer (Fe), du cuivre (Cu), du sodium (Na), du zinc (Zn), du bore (B), du molybdène (Mo), du silicium (Si), du sélénium (Se) et du nickel (Ni). En particulier, la suspension colloïdale concentrée comprend au moins un élément nutritif des végétaux. En particulier, la suspension colloïdale concentrée comprend au moins un élément nutritif choisi dans le groupe formé du carbonate de manganèse (MnCO₃), du sulfate de manganèse (MnSO₄), de l'hydroxyde de magnésium (Mg(OH)₂), de l'oxyde de zinc (ZnO), de l'oxyde de calcium (CaO), de chélate de fer -notamment de chélate d'EDTA ou de chélate d'EDDHA (acide éthylènediamine-N,N'-bis (2-hydroxyphénylacétique) et de fer-, de chélate de cuivre -notamment de chélate d'EDTA et de cuivre-, de chélate de zinc -notamment de chélate d'EDTA et de zinc-, du sulfate de fer, du sulfate de cuivre, du sulfate de zinc et du pentoxyde de phosphore (P₂O₅).

La suspension colloïdale concentrée présente une concentration du solide à l'état divisé comprise entre 0,001 g/L et 2 Kg/L. En particulier, la suspension colloïdale concentrée de carbonate de manganèse (MnCO₃) présente une concentration de manganèse comprise entre 80 g/L et 600 g/L.

La suspension colloïdale concentrée est stable vis-à-vis de la sédimentation dans le temps -en particulier sur une durée de 12 mois- et est susceptible de pouvoir être conservée à une température comprise entre -50°C et +50°C sans altération de ses propriétés rhéologiques ni des propriétés stimulantes et/ou nutritives de la composition nutritive obtenue par dilution de la suspension colloïdale concentrée. Une telle suspension colloïdale concentrée peut donc être proposée à la vente, commercialisée et distribuée en conservant les propriétés stimulantes et/ou nutritives de la composition nutritive.

Avantageusement, la suspension colloïdale concentrée comprend entre 1 % et 5 % de cet ester carbonique de glycérol, entre 1 % et 20 % d'eau, entre 15 % et 50 % de glycérol et entre 10 % et 90 % du solide à l'état divisé comprenant au moins un élément nutritif des végétaux -notamment du carbonate de manganèse (MnCO₃) et/ou du sulfate de manganèse (MnSO₄)-, les pourcentages représentant des proportions massiques.

Avantageusement et selon l'invention, la suspension colloïdale concentrée et la composition nutritive peuvent comprendre une pluralité d'esters carboniques de glycérol selon les revendications à et/ou une pluralité d'éléments nutritifs des végétaux.

Avantageusement et selon l'invention, la suspension colloïdale concentrée et la composition nutritive comprennent au moins cet ester carbonique de glycérol et au moins un solide à l'état divisé comprenant au moins un composé choisi dans le groupe formé des éléments nutritifs des végétaux, par exemple du carbonate de manganèse (MnCO₃) et/ou du sulfate de manganèse (MnSO₄).

Avantageusement, au moins un solide à l'état divisé est formé de particules solides présentant une taille moyenne comprise entre 1,5 µm et 2,5 µm, notamment de l'ordre de 2 µm.

Avantageusement, au moins un solide à l'état divisé est choisi dans le groupe formé des matériaux sensiblement insolubles dans l'eau et dans les esters carboniques de glycérol.

Avantageusement et selon l'invention, la suspension colloïdale concentrée et la composition nutritive comprennent à titre d'ester carbonique de glycérol au moins un ester carbonique linéaire de glycérol α/α'-acylé de formule (I), (I') ou (X).

Avantageusement et selon l'invention, la suspension colloïdale concentrée et la composition nutritive comprennent à titre d'ester carbonique de glycérol au moins un ester carbonique de glycérol de formule (I), (I'), (VIII), (IX) ou (X).

Avantageusement, au moins un ester carbonique de glycérol de la suspension colloïdale concentrée et de la composition nutritive est choisi dans le groupe formé :
- du carbonate cyclique de glycérol à six chaînons ;
- de l'ester carbonique cyclique de glycérol α/α'-acylé de formule (VIII) dans laquelle R1 est un groupe méthyle ;
- de l'ester carbonique cyclique de glycérol α/α'-acylé de formule (VIII) dans laquelle R1 est un groupe n-hexyle ;
- de l'ester carbonique cyclique de glycérol α/α'-acylé de formule (VIII) dans laquelle R1 est un groupe n-octyle ;
- de l'ester carbonique cyclique de glycérol α/α'-acylé de formule (VIII) dans laquelle R1 est un groupe 9-ène-decyle ;
- de l'ester carbonique cyclique de glycérol α/α'-acylé de formule (VIII) dans laquelle R1 est un groupe oléyle ;
- des esters carboniques cycliques de glycérol β-acylés de formule (IX) ;
- des esters carboniques linéaires de glycérol de formule (X).

Une suspension colloïdale selon l'invention peut comprendre optionnellement un excipient phyto-acceptable autre qu'un ester carbonique de glycérol.

Avantageusement et selon l'invention, la suspension colloïdale concentrée et la composition nutritive comprennent aussi au moins l'un des corps gras choisis dans le groupe formé de l'huile de jojoba (dans une proportion comprise entre 0,1 % et 5 % -notamment de l'ordre de 1 %-), des triglycérides -notamment des triglycérides oléiques- (dans une proportion comprise entre 0,1 % et 5 %) et des sels de l'acide oléique (dans une proportion comprise entre 0,5 % et 5 %).

On extrait l'huile de jojoba des graines de *Simmondsia chinensis* sous la forme d'une composition d'esters d'acides gras essentiellement insaturés (notamment d'acide eïcosénoïque, d'acide docosénoïque et d'acide oléique) et d'alcool gras, chaque ester présentant entre 36 et 46 atomes de carbone.

Avantageusement, il est possible que la suspension colloïdale concentrée et/ou composition nutritive comprennent aussi au moins un composé minéral ou organique choisi dans le groupe formé des dispersants, des émulsifiants, des stabilisants, des gélifiants.

Avantageusement et selon l'invention, la suspension colloïdale concentrée et la composition nutritives selon l'invention comprennent :
- au moins un ester carbonique de glycérol selon les revendications ;
- du glycérol ;
- au moins un composé choisi dans le groupe formé du sulfate de zinc (ZnSO₄), du stéarate de zinc (Zn(C₁₈H₃₅O₂)₂), du sulfate de fer (FeSO₄), du phosphate ferrique (FePO₄), du sulfate de manganèse (MnSO₄), de l'oxyde de zinc (ZnO), du carbonate de calcium (Ca₂CO₃), du carbonate de sodium (Na₂CO₃) et du sulfate de sodium (Na₂SO₄), et ;
- au moins un solide à l'état divisé comprenant au moins un composé choisi dans le groupe formé des éléments nutritifs de végétaux, notamment du carbonate de manganèse (MnCO₃) et/ou du sulfate de manganèse (MnSO₄).

L'invention concerne en particulier des adjuvants de vectorisation pour des compositions nutritives de végétaux. Un tel adjuvant permet non seulement le maintien en suspension de particules de matériau solide à l'état divisé dans une suspension colloïdale concentrée et dans la composition nutritive, mais permet aussi une libération contrôlée des éléments nutritifs, par exemple une libération rapide d'élément(s) nutritif(s) pendant un à quatre jours suivant l'application ou une libération prolongée -notamment une libération retardée-d'élément(s) nutritif(s), selon le choix des esters carboniques de glycérol. Un tel adjuvant sous forme de suspension colloïdale concentrée présente une viscosité qui peut être ajustée, et permet aussi d'ajuster la viscosité de la composition nutritive. Un tel adjuvant est en outre thixotrope. Il peut être stocké à basse température sans geler. Un tel adjuvant est miscible dans l'eau et peut être dilué dans l'eau de façon extemporanée préalablement à son application sur des végétaux.

L'invention s'étend aussi à une composition de traitement de végétaux comprenant au moins un ester carbonique de glycérol selon les revendications -notamment au moins un ester carbonique de glycérol de formule (I), (I'), (VIII), (IX) ou (X)- et au moins un agent actif autre qu'un ester carbonique de glycérol et choisi dans le groupe formé des agents actifs sur la germination de semences, des agents de fertilisation et des nutriments. Une composition de traitement de végétaux peut optionnellement comprendre un excipient phyto-acceptable autre qu'un ester carbonique de glycérol.

L'invention concerne également un procédé de traitement de végétaux, les utilisations d'esters carboniques de glycérol selon les revendications à titre d'intrant en agriculture, une suspension colloïdale concentrée et une composition de traitement de végétaux comprenant au moins un ester carbonique de glycérol selon les revendications caractérisés en combinaison par tout ou partie des caractéristiques mentionnées ci-dessus ou ci-après.

D'autres buts, caractéristiques et avantages de l'invention apparaîtront à la lecture de la description suivante et des exemples de mise en oeuvre de l'invention donnés uniquement à titre non limitatif et dans lesquels :
- la figure 1 est une représentation graphique de l'évolution de la teneur foliaire en manganèse de plants de soja en culture après application de compositions nutritives comprenant au moins un ester carbonique cyclique de glycérol exclu de l'invention et du carbonate de manganèse ;
- la figure 2 est une représentation graphique de l'évolution de la teneur foliaire en manganèse de plants de soja en culture après application de compositions nutritives comprenant au moins un ester carbonique cyclique de glycérol α/α'-acylé selon l'invention et du carbonate de manganèse ;
- la figure 3 est une représentation graphique de l'évolution de la teneur foliaire en manganèse de plants de soja en culture après application de compositions nutritives comprenant au moins un ester carbonique linéaire de glycérol selon l'invention et du carbonate de manganèse,
- la figure 4 est un spectre de masse réalisé sur un milieu de réaction obtenu par mise en oeuvre d'un procédé d'oligomérisation de l'ester carbonique de glycérol α/α'-acétylé (ECG-C2) tel que décrit à l'exemple 13.

Selon la présente invention, on utilise au moins un ester carbonique de glycérol selon les revendications.

Le carbonate de glycérol (CAS 931-40-8) cyclique à 5 chainons ou 4-(hydroxyméthyl)-1,3-dioxolan-2-one est décrit par exemple dans FR 2 733 232.

Les esters carboniques cycliques de glycérol α/α'-acylés de formule générale (VIII) peuvent être obtenus par toute méthode appropriée. En particulier, pour une utilisation selon l'invention, un ester carbonique cyclique de glycérol α/α'-acylé de formule (VIII) peut, par exemple, être obtenu par un procédé de synthèse dans lequel on réalise une acylation d'un ester carbonique cyclique de glycérol. Par exemple, on réalise une telle acylation par addition goutte à goutte et sous agitation d'ester carbonique cyclique de glycérol dans un milieu liquide comprenant une quantité d'au moins un acide gras (par exemple non limitatif de l'acide heptanoïque, de l'acide nonanoïque, de l'acide undécylénique ou de l'acide oléique) et de l'acide 4-méthylbenzènesulfonique (CAS n° 6192-52-5, acide para-toluène-sulfonique, ApTs) porté à une température de l'ordre de 110°C, sous une pression de l'ordre de 800 hPa. On maintient le mélange réactionnel obtenu sous agitation à 110°C et sous 900 hPa pendant de l'ordre de 3 heures.

On synthétise, par exemple, de cette façon l'ester carbonique cyclique de glycérol α/α'-heptanoylé (ECG-C₇), l'ester carbonique cyclique de glycérol α/α'-nonanoylé (ECG-C₉), l'ester carbonique cyclique de glycérol α/α'-undécylènoylé (ECG-C_{11:1}), l'ester carbonique cyclique de glycérol α/α'-oléylé (ECG-C_{18:1}).

Il est aussi possible d'obtenir un tel ester carbonique cyclique de glycérol α/α'-acylé de formule (VIII) par addition d'un anhydride à du carbonate cyclique de glycérol en présence d'une résine échangeuse d'ions.

On obtient par exemple l'ester carbonique cyclique de glycérol α/α'-acétylé ou acétate de carbonate de glycérol (ECG-C₂) par addition d'anhydride acétique sur du glycérol en présence d'une résine échangeuse d'ions à titre de catalyseur et en maintenant la température du milieu réactionnel à 50°C sous agitation mécanique pendant de l'ordre de 4 heures. On purifie l'acétate de carbonate de glycérol par la technique du film mince à la température de 170°C et sous pression réduite.

Des esters carboniques linéaires de glycérol α/α'-acylés et des esters carboniques linéaires de glycérol β-acylés peuvent être obtenus par un procédé particulier découlant du procédé général décrit ci-après.

On obtient des esters carboniques linéaires de glycérol α/α'-acylés -notamment des oligomères d'esters carboniques linéaires de glycérol α/α'-acylés- de formule générale (X) par synthèse à partir d'au moins un ester carbonique cyclique de glycérol α/α'-acylé de formule (VIII) générale, d'au moins un amorceur organique choisi dans le groupe formé des composés organiques hydroxylés -notamment des polyols, en particulier du glycérol- et d'au moins un catalyseur métallique choisi, par exemple, dans le groupe formé du sulfate de zinc (ZnSO₄), du stéarate de zinc (Zn(C₁₈H₃₅O₂)₂), du sulfate de fer (FeSO₄), du phosphate ferrique (FePO₄), du sulfate de manganèse (MnSO₄), de l'oxyde de zinc (ZnO), du carbonate de calcium (Ca₂CO₃), du carbonate de sodium (Na₂CO₃) et du sulfate de sodium (Na₂SO₄). Dans un tel procédé de synthèse, on mélange les esters carboniques cycliques de glycérol α/α'-acylé(s) de formule (VIII), l'amorceur organique et le catalyseur métallique dans un réacteur que l'on clôt hermétiquement et que l'on porte à une température comprise entre 150°C et 220°C et de façon à placer le mélange liquide sous une pression, dite pression autogène, supérieure ou égale à la pression atmosphérique. Dans un tel procédé de synthèse, on maitrise la polymérisation des esters carboniques cyclique(s) de glycérol α/α'-acylé(s) de formule (VIII) générale et la formation d'esters carboniques linéaires de glycérol α/α'-acylés de formule (X) générale en contrôlant la pression autogène générée par chauffage du milieu réactionnel hermétiquement clos.

Les masses molaires moyennes des esters carboniques linéaires de glycérol acylés susceptible d'être obtenus par le procédé ci-dessus sont décrites au tableau 1 ci-après.

### EXEMPLE 1 - Synthèse d'esters carboniques de glycérol cycliques α/α'-acylés.

On réalise la synthèse d'esters carboniques de glycérol cycliques α/α'-acylés, en particulier d'ester carbonique cyclique de glycérol α/α'-heptanoïque (ECG-C₇), d'ester carbonique cyclique de glycérol α/α'-nonanoïque (ECG-C₉), d'ester carbonique cyclique de glycérol α/α'-undécylénoïque (ECG-C_{11:1}) et d'ester carbonique cyclique de glycérol α/α'-oléique (ECG-C_{18:1}) par estérification du carbonate de glycérol cyclique α/α'-hydroxylé avec un acide gras correspondant.

Dans un réacteur de 500 mL équipé d'un dispositif d'agitation mécanique, d'un dispositif de mise sous pression réduite et d'un dispositif "Dean-Stark" d'élimination de l'eau formée, on place 1,64 moles d'acide gras et 0,0078 moles d'acide 4-méthylbenzènesulfonique (CAS n° 6192-52-5, acide para-toluène-sulfonique, ApTs). On porte la température du mélange à la température de 110°C sous pression réduite de 800 hPa pendant une durée de 15 min. On ajoute ensuite goutte à goutte dans le réacteur 0,84 moles de carbonate de glycérol cyclique α/α'-hydroxylé sous agitation mécanique à 800 rotations par minutes (rpm) pendant 15 min. On place le réacteur dans un bain d'huile porté à la température de 110°C et sous agitation mécanique (800 rpm) pendant 3 heures.

### EXEMPLE 2 - Purification d'esters carboniques cycliques de glycérol α/α'-acylés.

On dilue le milieu réactionnel dans 150 mL d'éther éthylique et on place le mélange obtenu dans une ampoule à décanter de 1 L. On lave le mélange successivement avec 4 volumes d'eau saturée en NaCl jusqu'à neutralité de la phase aqueuse. La phase organique lavée est séchée sur du sulfate de magnésium, puis est séparée du sulfate de magnésium hydraté par filtration. L'éther de la phase organique est éliminé par évaporation sous pression réduite. On obtient une masse de produit sec de 277g. L'ester carbonique cyclique de glycérol α/α'-acylé est séparé des acides gras en excès par distillation en film mince sous pression réduite (0,6 hPa) à une température inférieure à la température d'ébullition de l'acide gras sous cette pression réduite et inférieure à 155°C. On obtient le carbonate cyclique de glycérol α/α'-acylé dont la pureté évaluée par chromatographie en phase gazeuse est comprise entre 85 % et 95 %.

### EXEMPLE 3 - Synthèse de l'ester carbonique cyclique de glycérol α/α'-acétylé (ECG-C₂).

Dans un ballon tricol en verre de 2 L équipé d'un agitateur mécanique et d'un dispositif "Dean-Stark" d'élimination de l'eau formée comprenant un réfrigérant et placé dans un bain d'huile, on introduit 472 g de carbonate cyclique de glycérol (4-(hydroxyméthyle)-1,3-dioxolan-2-one, CAS 931-40-8) et 4 g de résine Lewatit K2431. On ajoute 6 moles d'anhydride acétique goutte à goutte dans le réacteur de façon à contrôler et maintenir la température du réacteur à 50°C sous agitation mécanique de 800 rpm pendant 4 heures.

On élimine l'excès d'anhydride acétique par évaporation à la température de 60°C et sous pression réduite de 55 hPa. On purifie l'ester carbonique linéaire de glycérol α/α'-acétylé par la technique du film mince conduite dans un évaporateur/séparateur à la température de 170°C et sous pression réduite de 0,33 hPa. On obtient l'ester carbonique cyclique de glycérol α/α'-acétylé dont la pureté évaluée par chromatographie en phase gazeuse est comprise entre 85 % et 98 %.

Les structures chimiques des esters carboniques de glycérol cycliques α/α'-acylés synthétisés dans les exemples 1, 2 et 3 sont confirmées par spectrométrie de masse, par RMN du proton, par RMN du ¹³C et par spectroscopie infra-rouge à transformée de Fourrier. La pureté des esters carboniques de glycérol cycliques α/α'-acylés et la masse de l'ion moléculaire sont données au tableau 1 ci-après.

**Tableau 1**

| | Pureté, % | Spectrométrie de masse, m/z |
|---|---|---|
| ECG-C₂ | 98 | 160,1 |
| ECG-C₇ | 94 | 230,2 |
| ECG-C₉ | 95 | 258,3 |
| ECG-C_{11:1} | 85 | 284,3 |
| ECG-C_{18:1} | 96 | 382,5 |

### EXEMPLE 4 - Utilisation de carbonate cyclique de glycérol (exclu de l'invention) dans une composition nutritive pour la culture de soja.

On mélange du carbonate cyclique de glycérol (CG) de formule (VI), du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,2 % de carbonate cyclique de glycérol, de l'ordre de 27,3 % de glycérol, de l'ordre de 10,5 % d'eau et de l'ordre de 58,7 % de MnCO₃. On obtient une suspension colloïdale concentrée (CG-Mn500) de MnCO₃ qui est stable dans le temps et dont les particules ne sédimentent pas.

On dilue cette suspension colloïdale concentrée dans l'eau et on applique par nébulisation (à l'aide d'un pulvérisateur) cette composition nutritive sur la culture à raison de 500 grammes de carbonate de manganèse par hectare de culture.

Une telle composition permet une application foliaire de MnCO₃ sur soja et le maintien d'une teneur foliaire en carbonate de manganèse (MnCO₃) élevée de l'ordre de 110 à 160 ppm pendant une durée de l'ordre 7 à 8 jours. À titre de contrôle négatif (Mn500), on applique sur une culture de soja une suspension de carbonate de manganèse dans l'eau à raison de 500 g de carbonate de manganèse (MnCO₃) par hectare de culture. Dans les conditions du contrôle négatif, la teneur foliaire en manganèse (Mn) de la modalité soja traitée reste faible, inférieure à 50 ppm et de l'ordre de 30 ppm pendant 8 jours. Cette teneur foliaire en MnCO₃ du contrôle négatif est identique à la teneur foliaire en manganèse obtenue par traitement d'une culture de céréale avec une quantité comparable d'eau ("eau").

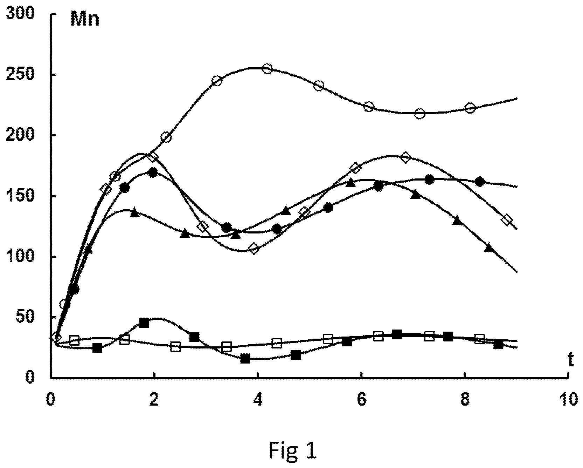

Les résultats sont présentés en figures 1, 2 ou 3 dans lesquelles :
- le contrôle "eau" est identifié par un carré plein (■) ;
- le contrôle négatif "Mn500" est identifié par un carré vide (□) ;
- l'essai carbonate cyclique de glycérol "CG Mn500" est identifié par un triangle plein (▲).

### EXEMPLE 5 - Utilisation d'acétate de carbonate cyclique de glycérol (ECG-C₂) à titre d'adjuvant dans une composition nutritive pour la culture de soja.

On mélange de l'acétate de carbonate cyclique de glycérol (ECG-C₂), du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,25 % d'acétate de carbonate cyclique de glycérol, de l'ordre de 27,3 % de glycérol, de l'ordre de 10,5 % d'eau et de l'ordre de 58,7 % de MnCO₃. On obtient une suspension colloïdale concentrée de MnCO₃ qui est stable dans le temps et dont les particules ne sédimentent pas.

On dilue cette suspension colloïdale concentrée dans l'eau et on applique par nébulisation de la composition nutritive diluée sur la culture de soja à raison de 500 grammes de carbonate de manganèse par hectare de culture.

Une telle composition nutritive permet une application foliaire de MnCO₃ sur soja et le maintien d'une teneur foliaire en carbonate de manganèse (MnCO₃) élevée et comprise entre 100 et 180 ppm pendant une durée supérieure à 8 jours. Les résultats sont présentés en figure 1 (ECG-C₂ est identifié par un losange vide (◊)).

### EXEMPLE 6 - Utilisation de carbonate cyclique de glycérol (exclu de l'invention) à titre d'adjuvant dans une composition nutritive.

On mélange du carbonate cyclique de glycérol de formule (VI), du glycérol, de l'eau, du carbonate de manganèse (MnCO₃) et du sulfate de manganèse (MnSO₄), MnCO₃ et MnSO₄ étant sous forme de solides à l'état divisé et dont les particules solides présentent une taille moyenne de l'ordre de 2 µm. Les proportions massiques sont de l'ordre de 1,2 % d'acétate de carbonate cyclique de glycérol, de l'ordre de 1,97 % de glycérol, de l'ordre de 25,6 % d'eau, de l'ordre de 55,2 % de MnCO₃ et de l'ordre de 12,1 % de MnSO₄. On obtient une suspension colloïdale concentrée de MnCO₃ et de MnSO₄ qui est stable dans le temps et dont les particules ne sédimentent pas.

Cette suspension peut être diluée pour obtenir une composition nutritive, ou ajoutée à une formulation de composition nutritive, notamment pour la culture de soja.

### EXEMPLE 7 - Utilisation de carbonate cyclique de glycérol (exclu de l'invention) à titre d'adjuvant dans une composition nutritive.

On mélange du carbonate cyclique de glycérol de formule (VI), du glycérol, de l'eau et du carbonate de manganèse (MnCO₃) sous forme d'un solide à l'état divisé et dont les particules solides présentent une taille moyenne de l'ordre de 2 µm. Les proportions massiques sont de l'ordre de 1,22 % d'acétate de carbonate cyclique de glycérol, de l'ordre de 8,15 % de glycérol, de l'ordre de 20,38 % d'eau et de l'ordre de 67,61 % de MnCO₃. On obtient une suspension colloïdale concentrée de MnCO₃ qui est stable dans le temps et dont les particules ne sédimentent pas.

Cette suspension peut être diluée pour obtenir une composition nutritive, ou ajoutée à une formulation de composition nutritive, notamment pour la culture de soja.

### EXEMPLE 8 - Utilisation d'acétate de carbonate cyclique de glycérol (ECG-C₂) à titre d'adjuvant dans une composition nutritive pour la culture de soja.

On mélange de l'acétate de carbonate cyclique de glycérol (ECG-C₂), des triglycérides oléiques (THO), un sel d'acide oléique, du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,5 % d'acétate de carbonate cyclique de glycérol, de l'ordre de 39,5 % de glycérol, de l'ordre de 7,5 % d'eau, de l'ordre de 1 % de triglycéride oléique, de l'ordre de 0,5 % d'oléate, et de l'ordre de 50 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par nébulisation cette composition nutritive diluée sur une culture de soja à raison de 500 grammes de carbonate de manganèse par hectare de culture.

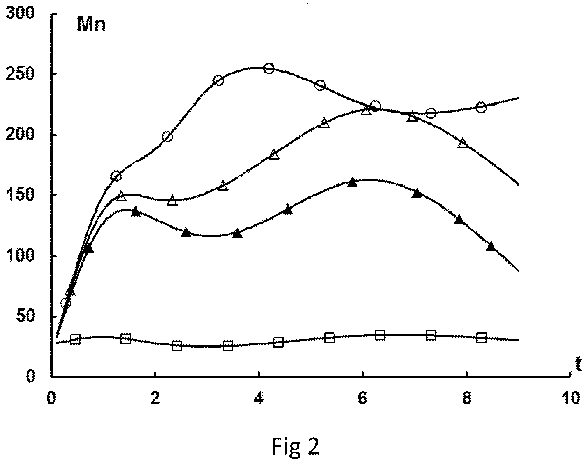

Une telle composition nutritive permet une application foliaire de MnCO₃ sur soja et le maintien d'une teneur foliaire en manganèse élevée et comprise entre 150 et 220 ppm pendant une durée supérieure à 8 jours. Les résultats sont présentés en figure 2 (THO/ECG-C₂ est identifié par un triangle vide (Δ).

### EXEMPLE 9 - Utilisation d'heptanoate de carbonate cyclique de glycérol (ECG-C₇) à titre d'adjuvant dans une composition nutritive pour la culture de soja.

On mélange de l'heptanoate de carbonate cyclique de glycérol (ECG-C₇), du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,23 % d'heptanoate de carbonate cyclique de glycérol, de l'ordre de 24,6 % de glycérol, de l'ordre de 14,4 % d'eau et de l'ordre de 57,5 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par nébulisation cette composition nutritive diluée sur la culture de soja à raison de 500 grammes de carbonate de manganèse par hectare de culture.

Une telle composition nutritive permet une application foliaire de MnCO₃ sur soja, ne produit pas de brulure sur les feuilles de soja et permet le maintien d'une teneur foliaire en manganèse élevée.

### EXEMPLE 10 - Utilisation de nonanoate de carbonate cyclique de glycérol (ECG-C₉) à titre d'adjuvant dans une composition nutritive pour la culture de soja.

On mélange du nonanoate de carbonate cyclique de glycérol (ECG-C₉), du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,23 % de nonanoate de carbonate cyclique de glycérol, de l'ordre de 24,6 % de glycérol, de l'ordre de 14,4 % d'eau et de l'ordre de 57,5 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par nébulisation cette composition nutritive diluée sur la culture de soja à raison de 500 grammes de carbonate de manganèse par hectare de culture.

Une telle composition nutritive permet une application foliaire de MnCO₃ sur soja, ne produit pas de brûlure sur les feuilles de soja et permet le maintien d'une teneur foliaire en manganèse élevée.

### EXEMPLE 11 - Utilisation d'undécylénate de carbonate cyclique de glycérol (ECG-C_{11:1}) à titre d'adjuvant dans une composition nutritive pour la culture de soja.

On mélange de l'undécylénate de carbonate cyclique de glycérol (ECG-C_{11:1}), du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,23 % d'undécylénate de carbonate cyclique de glycérol, de l'ordre de 24,65 % de glycérol, de l'ordre de 14,4 % d'eau et de l'ordre de 57,5 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par nébulisation cette composition nutritive diluée sur la culture de soja à raison de 500 grammes de carbonate de manganèse par hectare de culture.

Une telle composition nutritive permet une application foliaire de MnCO₃ sur soja et le maintien d'une teneur foliaire en carbonate de manganèse (MnCO₃) élevée et comprise entre 120 et 160 ppm pendant une durée supérieure à 8 jours. Les résultats sont présentés en figure 1 (ECG-C_{11:1} est identifié par un cercle plein (●)).

### EXEMPLE 12 - Utilisation d'oléate de carbonate cyclique de glycérol (ECG-C_{18:1}) à titre d'adjuvant dans une composition nutritive pour la culture de soja.

On mélange de l'oléate de carbonate cyclique de glycérol (ECG-C_{18:1}), du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,23 % d'oléate de carbonate cyclique de glycérol, de l'ordre de 24,65 % de glycérol, de l'ordre de 14,4 % d'eau et de l'ordre de 57,5 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par nébulisation cette composition nutritive diluée sur la culture de soja à raison de 500 grammes de carbonate de manganèse par hectare de culture.

Une telle composition nutritive permet une application foliaire de MnCO₃ sur une culture de soja, l'augmentation de la teneur foliaire en manganèse jusqu'à quatre jours après son application pour atteindre une teneur foliaire maximale en manganèse supérieure à 250 ppm et le maintien d'une teneur foliaire en manganèse comprise entre 200 et 250 ppm pendant une durée supérieure à 8 jours. Les résultats sont présentés en figure 1 (ECG-C_{11:1} est identifié par un cercle vide (○)).

### EXEMPLE 13 - Synthèse d'oligomères de glycérol acétylés (OECG-C₂) par oligomérisation de l'ester carbonique cyclique de glycérol α/α'-acétylé (ECG-C₂).

On met en contact 21,25 g d'ECG-C₂ tel qu'obtenu à l'exemple 3, 125 mg de stéarate de zinc (Zn(C₁₈H₃₅O₂)₂ à titre de catalyseur et 3,75 g de glycérol dans un réacteur que l'on clôt hermétiquement. On chauffe ensuite le milieu de réaction dans le réacteur hermétiquement clos de façon à atteindre la température de 160°C sous pression autogène, puis on ramène la pression à la pression atmosphérique et on maintient la température de 160°C pendant 2 heures à la pression atmosphérique. Le taux de conversion de l'ECG-C₂ est de 98 %. L'analyse en chromatographie par perméation de gel démontre la formation d'oligomères de masse moléculaire en nombre de 714 g/mole, 335 g/mole, 206 g/mole, 139 g/mole et 92 g/mole.

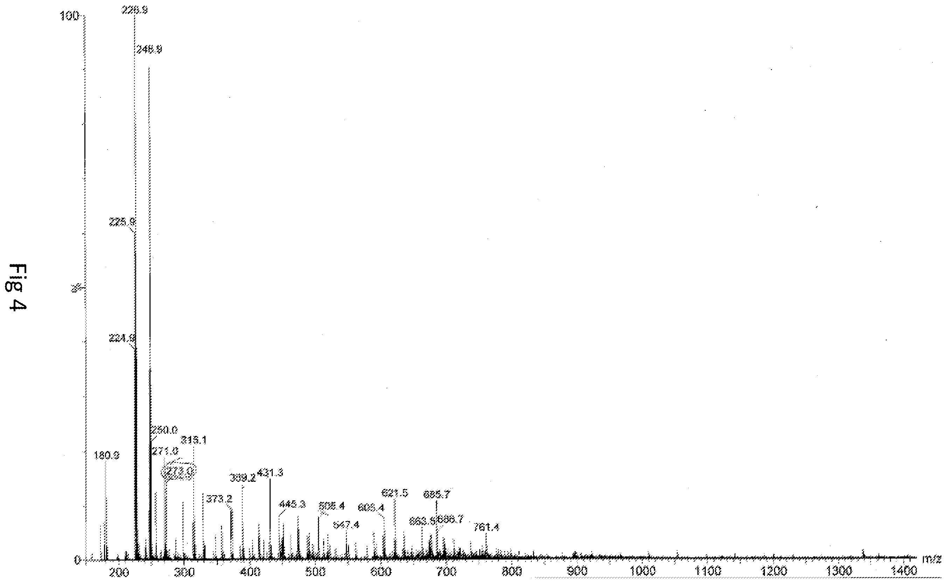

Un exemple de spectre de masse réalisé sur un milieu de réaction obtenu par mise en oeuvre d'un procédé d'oligomérisation de l'ester carbonique de glycérol α/α'-acétylé (ECG-C2) tel que décrit à l'exemple 13 est représenté en figure 4. Sont détectés des signaux correspondant à des ions moléculaires et fragments de valeurs m/z comprises entre 180,9 et 761,4 et correspondant à des oligomères de formules (A), (B), (C) et (D) suivantes : 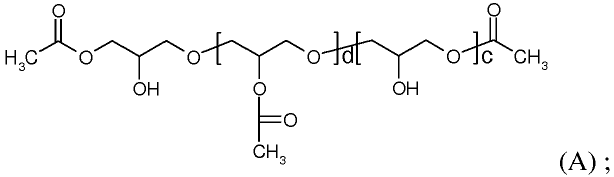 dans laquelle c peut prendre la valeur 1, 2 ou 4 et d peut prendre la valeur 1, 2, 3 ou 4 ; 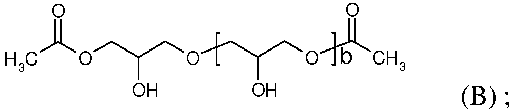 dans laquelle b peut prendre la valeur 1, 2, ou 3; 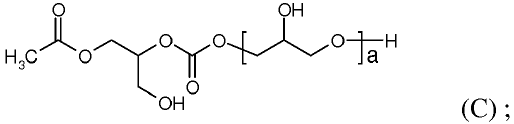 dans laquelle a est 1, 2, 3, 4, 5, 6, 7 ou 8, et ; 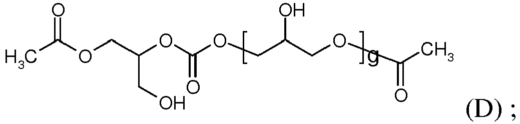 dans laquelle g peut prendre la valeur 1, 2, 3, 4, 5, 6, 7, 8, 9 ou 10.

### EXEMPLE 14 - Synthèse d'oligomères de glycérol acétylés (OECG-C₂) par oligomérisation de l'ester carbonique cyclique de glycérol α/α'-acétylé (ECG-C₂).

On met en contact 21,25 g d'ECG-C₂ tel qu'obtenu à l'exemple 3, 125 mg de sulfate de zinc (ZnSO₄) à titre de catalyseur et 3,75 g de glycérol à titre d'amorceur organique dans un réacteur que l'on clôt hermétiquement. On chauffe ensuite le milieu de réaction dans le réacteur hermétiquement clos de façon à atteindre la température de 160°C sous pression autogène, puis on ramène la pression à la pression atmosphérique et on maintient la température de 160°C pendant 2 heures à la pression atmosphérique. Le taux de conversion de l'ECG-C₂ est de 66 %. L'analyse en chromatographie par perméation de gel démontre la formation d'oligomères de masse moléculaire en nombre de 389 g/mole, 159 g/mole et 83 g/mole.

### EXEMPLE 15 - Utilisation d'une composition comprenant des oligomères de glycérol acétylés (OECG-C₂) à titre d'adjuvant dans une composition nutritive pour la culture de soja.

On mélange une composition comprenant des oligomères (OECG-C₂) telle que décrite à l'exemple 13, du glycérol, de l'eau et du carbonate de manganèse (MnCO₃) sous forme de solide à l'état divisé et dont les particules solides présentent une taille moyenne de l'ordre de 2 µm. Les proportions massiques dans ce mélange sont de l'ordre de 1,25 % d'OECG-C₂, de l'ordre de 27,72 % de glycérol, de l'ordre de 10,41 % d'eau et de l'ordre de 58,33 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par nébulisation cette composition nutritive diluée sur la culture de soja à raison de 500 grammes de carbonate de manganèse par hectare de culture.

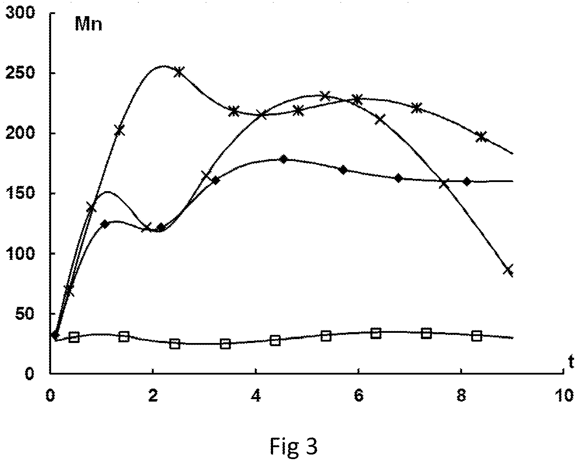

Une telle composition nutritive permet une application foliaire de MnCO₃ sur soja, ne produit pas de brûlure sur les feuilles de soja et permet le maintien d'une teneur foliaire en manganèse élevée pendant au moins 8 jours. Les résultats sont présentés en figure 3 (le traitement OECG-C₂ est identifié par un losange plein (◆)).

### EXEMPLE 16 - Utilisation d'une composition comprenant des oligomères de glycérol (OECG-C₂) à titre d'adjuvant dans une composition nutritive pour la culture de soja.

On mélange une composition comprenant des oligomères (OECG-C₂) telle que décrite à l'exemple 13, de l'acétate de carbonate cyclique de glycérol (ECG-C₂) tel que décrit à l'exemple 3, du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,25 % d'OECG-C₂, de l'ordre de 0,2 % d'ECG-C₂, de l'ordre de 27,72 % de glycérol, de l'ordre de 10,41 % d'eau et de l'ordre de 58,33 % de MnCO₃. On obtient une suspension colloïdale concentrée de MnCO₃ qui est stable dans le temps et dont les particules de MnCO₃ ne sédimentent pas. Une telle suspension colloïdale concentrée comprend en outre du stéarate de zinc.

On dilue cette suspension colloïdale concentrée dans l'eau et on applique par nébulisation cette composition nutritive diluée sur la culture de soja à raison de 500 grammes de carbonate de manganèse par hectare de culture.

Une telle composition nutritive permet une application foliaire de MnCO₃ sur soja, l'augmentation de la teneur foliaire en manganèse dès le premier jour suivant l'application et jusqu'à quatre jours après son application pour atteindre une teneur foliaire en manganèse de l'ordre de 180 ppm et le maintien d'une teneur foliaire en manganèse supérieure à 150 ppm pendant une durée supérieure à 8 jours.

### EXEMPLE 17 - Utilisation d'une composition comprenant des oligomères de glycérol (OECG-C₂) à titre d'adjuvant dans une composition nutritive pour la culture de soja.

On mélange une composition comprenant des oligomères (OECG-C₂) telle que décrite à l'exemple 14, de l'acétate de carbonate cyclique de glycérol (ECG-C₂) tel que décrit à l'exemple 3, du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 1,25 % d'OECG-C2, de l'ordre de 0,55 % d'ECG-C₂, de l'ordre de 27,72 % de glycérol, de l'ordre de 10,41 % d'eau et de l'ordre de 58,33 % de MnCO₃. On obtient une suspension colloïdale concentrée de MnCO₃ qui est stable dans le temps et dont les particules de MnCO₃ ne sédimentent pas. Une telle suspension colloïdale concentrée comprend en outre du stéarate de zinc.

On dilue cette suspension colloïdale concentrée dans l'eau et on applique la composition nutritive diluée sur la culture de soja par nébulisation à raison de 500 grammes de carbonate de manganèse par hectare de culture.

Une telle composition nutritive permet une application foliaire de MnCO₃ sur soja, l'augmentation de la teneur foliaire en manganèse dès le premier jour suivant l'application et jusqu'à quatre jours après son application pour atteindre une teneur foliaire en manganèse de l'ordre de 180 ppm et le maintien d'une teneur foliaire en manganèse supérieure à 150 ppm pendant une durée supérieure à 8 jours.

### EXEMPLE 18 - Synthèse d'oligomères glycérol heptanoylés (OECG-C₇) par oligomérisation de l'ester carbonique cyclique de glycérol α/α'-heptanoylé (ECG-C₇).

On met en contact 20 g d'ECG-C₇ tel qu'obtenu aux exemples 1 et 2, 113 mg de sulfate de zinc (ZnSO₄) à titre de catalyseur et 2,42 g de glycérol à titre d'amorceur organique dans un réacteur que l'on clôt hermétiquement. On chauffe ensuite le milieu de réaction dans le réacteur hermétiquement clos de façon à atteindre la température de 200°C sous pression autogène, puis on ramène la pression à la pression atmosphérique et on maintient la température de 200°C pendant 2 heures à la pression atmosphérique. Le taux de conversion de l'ECG-C₇ est de 64 %. L'analyse en chromatographie par perméation de gel démontre la formation d'oligomères de masse moléculaire en nombre de 639 g/mole, 420 g/mole, 252 g/mole et 96 g/mole.

### EXEMPLE 19 - Utilisation d'une composition nutritive comprenant des oligomères de carbonate linéaire de glycérol (OECG-C₇) pour la culture de soja.

On mélange une composition comprenant des oligomères (OECG-C₇) telle que décrite à l'exemple 18, du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 0,625 % d'OECG-C₇, de l'ordre de 22,915 % de glycérol, de l'ordre de 15,84 % d'eau et de l'ordre de 58,33 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique la composition nutritive diluée sur la culture de soja par pulvérisation à raison de 500 grammes de carbonate de manganèse par hectare de culture. Cette composition nutritive permet une application foliaire de MnCO₃ sur soja et ne produit pas de brûlure sur les feuilles de soja.

### EXEMPLE 20 - Synthèse d'oligomères de glycérol nonanoylés (OECG-C₉) par oligomérisation de l'ester carbonique cyclique de glycérol α/α'-nonanoylé (ECG-C₉).

On met en contact 20 g d'ECG-C₉ tel qu'obtenu aux exemples 1 et 2, 110 mg de sulfate de zinc (ZnSO₄) à titre de catalyseur et 2,15 g de glycérol à titre d'amorceur organique dans un réacteur que l'on clôt hermétiquement. On chauffe ensuite le milieu de réaction dans le réacteur hermétiquement clos de façon à atteindre la température de 180°C sous pression autogène, puis on ramène la pression à la pression atmosphérique et on maintient la température de 180°C pendant 2 heures à la pression atmosphérique. Le taux de conversion de l'ECG-C₉ est de 88 %. L'analyse en chromatographie par perméation de gel démontre la formation d'oligomères de masse moléculaire en nombre de 992 g/mole, 541 g/mole, 303 g/mole et 90 g/mole.

### EXEMPLE 21 - Utilisation d'une composition nutritive comprenant des oligomères de glycérol (OECG-C₉) pour la culture de soja.

On mélange une composition comprenant des oligomères (OECG-C₉) telle que décrite à l'exemple 20, du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 0,625 % d'OECG-C₉, de l'ordre de 22,915 % de glycérol, de l'ordre de 15,84 % d'eau et de l'ordre de 58,33 % de MnCO₃. On obtient une suspension colloïdale concentrée de MnCO₃ qui est stable dans le temps et dont les particules de MnCO₃ ne sédimentent pas. On dilue cette suspension colloïdale concentrée dans l'eau et on applique la composition nutritive diluée sur la culture de soja par pulvérisation à raison de 500 grammes de carbonate de manganèse par hectare de culture. Cette composition nutritive permet une application foliaire de MnCO₃ sur soja sans produire de brûlure.

### EXEMPLE 22 - Synthèse d'oligomères glycérol undécylénoylés (OECG-C_{11:1}) par oligomérisation de l'ester carbonique cyclique de glycérol α/α'-undécylénoylé (ECG-C_{11:1}).

On met en contact 10 g d'ECG-C_{11:1} tel qu'obtenu aux exemples 1 et 2, 86 mg de sulfate de zinc (ZnSO₄) à titre de catalyseur et 1,3 g de glycérol à titre d'amorceur organique dans un réacteur que l'on clôt hermétiquement. On chauffe ensuite le milieu de réaction dans le réacteur hermétiquement clos de façon à atteindre la température de 190°C sous pression autogène, puis on ramène la pression à la pression atmosphérique et on maintient la température de 190°C pendant 2 heures à la pression atmosphérique. Le taux de conversion de l'ECG-C_{11:1} est de 98 %. L'analyse en chromatographie par perméation de gel démontre la formation d'oligomères de masse moléculaire en nombre de 7632 g/mole, 2113 g/mole, 1084 g/mole, 750 g/mole et 486 g/mole.

### EXEMPLE 23 - Utilisation d'une composition nutritive comprenant des oligomères glycérol (OECG-C_{11:1}) pour la culture de soja.

On mélange une composition comprenant des oligomères (OECG-C_{11:1}) telle que décrite à l'exemple 22, du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 0,625 % d'OECG-C_{11:1}, de l'ordre de 22,915 % de glycérol, de l'ordre de 15,84 % d'eau et de l'ordre de 58,33 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par pulvérisation la composition nutritive diluée sur la culture de soja à raison de 500 grammes de carbonate de manganèse par hectare de culture. Une telle composition nutritive ne produit pas de brûlure sur les feuilles de soja.

Une telle composition nutritive permet une augmentation de la teneur foliaire en MnCO₃ dès le premier jour suivant l'application et jusqu'à quatre jours après son application pour atteindre une teneur foliaire en manganèse de l'ordre de 260 ppm à deux jours après application et le maintien d'une teneur foliaire en manganèse supérieure à 200 ppm pendant une durée de 8 jours. Les résultats sont présentés en figure 3 (OECG-C_{11:1} est identifié par une étoile (∗)).

### EXEMPLE 24 - Synthèse d'oligomères de glycérol α/α'-oléylés (OECG-C_{18:1}) par oligomérisation de l'ester carbonique cyclique de glycérol α/α'-oléylé (ECG-C18:1).

On met en contact 10 g d'ECG-C_{18:1} tel qu'obtenu aux exemples 1 et 2, 50 mg de sulfate de zinc (ZnSO₄) à titre de catalyseur et 0,7 g de glycérol à titre d'amorceur organique dans un réacteur que l'on clôt hermétiquement. On chauffe ensuite le milieu de réaction dans le réacteur hermétiquement clos de façon à atteindre la température de 200°C sous pression autogène, puis on ramène la pression à la pression atmosphérique et on maintient la température de 200°C pendant 2 heures à la pression atmosphérique. Le taux de conversion de l'ECG-C_{18:1} est de 97 %. L'analyse en chromatographie par perméation de gel démontre la formation d'oligomères de masse moléculaire en nombre de 3724 g/mole, 1787 g/mole, 1169 g/mole, 613 g/mol et 94 g/mole.

### EXEMPLE 25 - Utilisation d'une composition nutritive comprenant des oligomères de glycérol (OECG-C_{18:1}) pour la culture de soja.

On mélange une composition comprenant des oligomères (OECG-C_{18:1}) telle que décrite à l'exemple 24, du glycérol, de l'eau et du carbonate de manganèse (MnCO₃) sous forme de solide à l'état divisé et dont les particules solides présentent une taille moyenne de l'ordre de 2 µm. Les proportions massiques sont de l'ordre de 0,625 % d'OECG-C_{18:1}, de l'ordre de 22,915 % de glycérol, de l'ordre de 15,84 % d'eau et de l'ordre de 58,33 % de MnCO₃. On obtient une suspension colloïdale concentrée de MnCO₃ qui est stable dans le temps et dont les particules de MnCO₃ ne sédimentent pas.

On dilue cette suspension colloïdale concentrée dans l'eau et on applique par pulvérisation la composition nutritive diluée sur la culture de soja à raison de 500 grammes de carbonate de manganèse par hectare. Cette composition nutritive ne produit pas de brûlure sur les feuilles de soja.

Une telle composition nutritive permet une augmentation de la teneur foliaire en manganèse dès le premier jour suivant l'application, puis une augmentation de la teneur foliaire en manganèse jusqu'à quatre jours après application pour atteindre une valeur maximale de 230 ppm et le déclin de la teneur foliaire en manganèse à une valeur inférieure à 100 ppm huit jours après application. Les résultats sont présentés en figure 3 (OECG-C_{18:1} est identifié par une croix (**×**)). Une telle composition nutritive permet donc une libération retardée du manganèse.

### EXEMPLE 26 - Essai comparatif d'un adjuvant selon l'invention avec la composition MANTRAC 500 sur l'absorption foliaire MnCO₃.

On prépare par broyage une poudre de carbonate de manganèse (MnCO₃, CAS 598-62-9) présentant une granulométrie moyenne sensiblement de l'ordre de 2 µm. On prépare une suspension colloïdale concentrée comprenant 58,7 % de cette poudre dans une composition liquide comprenant 1,5 % d'acétate de carbonate de glycérol (ECG-C₂, tel que décrit à l'exemple 3 et au tableau 1), 39,5 % de glycérol, 7,5 % d'eau, 1 % d'huile de jojoba, les pourcentages indiqués étant des pourcentages massiques. On dilue cette suspension colloïdale concentrée dans l'eau de façon à former une composition de traitement (Mn385) selon l'invention adaptée pour pouvoir appliquer, par pulvérisation sur la surface cultivée, une quantité de carbonate de manganèse de 385 g par hectare de surface cultivée.

On prépare aussi :
- une suspension colloïdale concentrée telle que décrite à l'exemple 4 et on dilue cette suspension colloïdale concentrée dans l'eau de façon à former une composition nutritive (Mn457) selon l'invention adaptée pour pouvoir appliquer par pulvérisation une quantité de carbonate de manganèse de 457 g par hectare de surface, et ;
- une suspension colloïdale concentrée telle que décrite à l'exemple 7 et on dilue cette suspension colloïdale concentrée dans l'eau de façon à former une composition nutritive (Mn509) selon l'invention adaptée pour pouvoir appliquer par pulvérisation une quantité de carbonate de manganèse de 509 g par hectare de surface cultivée.

Sur des lots de 4 parcelles de 16 m² de surface cultivées avec du blé tendre d'hiver (variété "Quality", ARVALIS) on applique par pulvérisation les compositions nutritives Mn385, Mn457 et Mn509 selon l'invention.

On réalise aussi en parallèle :
- sur un lot de 4 parcelles de 16 m², une culture de blé tendre d'hiver traitée par pulvérisation avec de l'eau à titre de contrôle négatif, et ;
- sur un lot de 4 parcelles de 16 m², une culture de blé tendre d'hiver traitée avec à raison de 500 g de MnCO₃ seul (sans adjuvant selon l'invention) par hectare à titre de traitement de contrôle, et ;
- sur un lot de 4 parcelles de 16 m², une culture de blé tendre d'hiver traitée, à titre de comparaison, avec une formulation de MANTRAC 500 à raison de 500 g de MnCO₃ par hectare.

Les résultats obtenus lors du traitement de contrôle par le MnCO₃ seul (sans adjuvant selon l'invention) ne se distinguent pas des résultats obtenus avec le contrôle négatif.

On mesure, un jour (J+1), sept jours (J+7), 14 jours (J+14) et 21 jours (J+21) après l'application, la teneur en manganèse retenu dans les feuilles de blé tendre traité. Pour ce faire on prélève, on lave les feuilles trois fois à l'eau pour éliminer le carbonate de manganèse non absorbé par la plante, puis on dose le manganèse absorbé dans les feuilles. Les résultats sont donnés au tableau 2 ci-après et exprimés en ppm (µg de manganèse par gramme de feuille).

**Tableau 2**

| | J+1 | J+7 | J+14 | J+21 |
|---|---|---|---|---|
| MnCO₃ seul | 84,83 +/- 28,01 | 74,83 +/- 19,14 | 112,50 +/- 45,35 | 95,43 +/- 5,95 |
| MANTRAC 500 | 228,07 +/-139,40 | 171,75 +/-13,38 | 157,55 +/-60,81 | 188,00 +/- 38,16 |
| Mn385 | 192,00 +/-58,92 | 138,75 +/-20,71 | 164,25 +/-27,18 | 153,00 +/-39,61 |
| Mn457 | 211,00 +/- 49,79 | 163,25 +/-44,66 | 177,25 +/-41,02 | 177,00 +/-18,25 |
| Mn509 | 329,00 +/-55,87 | 204,25 +/-44,62 | 180,75 +/- 67.31 | 210,33 +/-29,50 |

On observe que l'application de Mn509 permet d'augmenter la teneur foliaire en Mn par rapport au MANTRAC 500 à J+1, J+7, J+14 et J+21.

Le traitement Mn509 permet d'obtenir une teneur foliaire en MnCO₃ supérieure à la teneur foliaire en MnCO₃ obtenue lors du traitement par le MANTRAC 500.

L'utilisation de l'ECG-C₂ permet en outre d'obtenir une teneur foliaire en MnCO₃ sensiblement équivalente à la teneur foliaire en MnCO₃ obtenue avec le MANTRAC 500 mais avec une application de MnCO₃ (Mn457) à raison de 457 g MnCO₃ par hectare.

On mesure la cinétique d'assimilation journalière du MnCO₃ dans les feuilles. On observe, pour la composition Mn509, une assimilation journalière du manganèse qui augmente à partir du jour de traitement et qui se poursuit au moins jusqu'à 21 jours après l'application. Par comparaison, l'assimilation journalière par le blé tendre traité avec la composition Mn509 est deux fois plus importante que l'assimilation journalière par le blé tendre traité avec la composition MANTRAC 500.

Le traitement d'une céréale en culture avec la composition nutritive de MnCO₃ selon l'invention en application foliaire permet donc d'augmenter la teneur foliaire en manganèse de céréales traitées en comparaison à ces mêmes céréales en culture traitées avec une composition non conforme à l'invention (MANTRAC 500) comprenant la même quantité de manganèse (500 g/L), en particulier à 1 jour après le traitement, 7 jours après le traitement et 21 jours après le traitement.

### EXEMPLE 27 - Effet de l'application foliaire d'une composition nutritive de MnCO₃ selon l'invention sur la nutrition du blé en macroéléments (Nₜₒₜₐₗ, Cₜₒₜₐₗ, P, K, Ca, Mg) et en microéléments (Fe, Cu, Zn, B).

On prépare une suspension colloïdale concentrée comprenant 58,7 % de poudre de carbonate de manganèse (présentant une granulométrie moyenne sensiblement de l'ordre de 2 µm) dans une composition liquide comprenant 1,5 % d'acétate de carbonate de glycérol (ECG-C₂, tel que décrit à l'exemple 3 et au tableau 1), 39,5 % de glycérol, 7,5 % d'eau, 1 % d'huile de jojoba telle que décrite à l'exemple 26. On dilue cette suspension colloïdale concentrée dans l'eau de façon à former une composition nutritive selon l'invention que l'on applique sur une culture de blé.

L'analyse statistique, dite analyse à composante principale, de la mesure cinétique des teneurs foliaires en manganèse, en macroéléments et en microéléments à 1 jour (J+1), 7 jours (J+7), 14 jours (J+14) et 21 jours (J+21) après application de la composition nutritive de manganèse permet de montrer l'effet de la composition nutritive selon l'invention sur la nutrition globale de la plante. Le tableau 3 ci-après donne les valeurs des coefficients de corrélation de la teneur foliaire en manganèse par rapport aux valeurs des teneurs foliaires des différents macroéléments et microéléments. Les valeurs proches de 1 traduisent une synergie dans l'amélioration de la nutrition globale de la plante lors de la levée de la carence en manganèse chez le blé. Les valeurs négatives traduisent un effet antagoniste de la levée de la carence en manganèse sur la nutrition.

**Tableau 3**

| | J+1 | J+7 | J+14 | J+21 |
|---|---|---|---|---|
| Variables | Mn | Mn | Mn | Mn |
| Nₜₒₜₐₗ, % | -0.497 | -0.538 | 0.704 | 0.611 |
| Cₜₒₜₐₗ, % | -0.559 | 0.429 | 0.914 | -0.185 |
| P, % | 0.690 | 0.849 | -0.205 | 0.887 |
| K, % | -0.660 | 0.377 | -0.804 | -0.886 |
| Ca, % | 0.918 | 0.936 | 0.072 | 0.928 |
| Mg, % | 0.774 | 0.919 | -0.071 | 0.948 |
| Fe, % | 0.366 | -0.796 | 0.047 | -0.139 |
| Cu, % | 0.171 | 0.573 | 0.195 | 0.261 |
| Zn, % | 0.801 | 0.989 | -0.145 | -0.451 |
| B, % | 0.330 | 0.070 | 0.009 | -0.326 |

Le traitement nutritif d'une céréale en culture avec une composition nutritive comprenant du MnCO₃ en application foliaire permet d'équilibrer le statut nutritionnel et d'harmoniser la nutrition des macroéléments calcium, magnésium et phosphore et du microélément zinc dans un délai d'un jour à 21 jours après l'application et de la nutrition azotée à compter de la deuxième semaine après l'application (J+14).

### EXEMPLE 28 - Application foliaire d'une suspension de MnCO₃ selon l'invention sur la nutrition azotée du blé.

On analyse l'évolution de la teneur foliaire en azote dans les jours suivants le traitement de parcelles de blé traitées par application foliaire d'un contrôle négatif (CNNT) et des compositions de traitement MANTRAC500, Mn385, Mn457 et Mn509 de l'exemple 26. Le contrôle négatif (CNNT) montre une diminution continue de la teneur foliaire en azote. Cette diminution correspond à une remobilisation de l'azote hors du feuillage de la plante, en particulier lors de la phase de remplissage des graines. Le traitement MANTRAC500 entraine une augmentation de la teneur en azote dans les feuilles jusqu'à 7 jours après traitement, puis une diminution de l'azote foliaire en raison de la mobilisation de l'azote dans les graines. Le traitement Mn385 permet le maintien de la teneur foliaire en azote pendant 13 jours après le traitement, puis une diminution de l'azote foliaire. Le traitement Mn457 entraine une augmentation de la teneur en azote dans les feuilles jusqu'à 11 jours après traitement, puis une diminution de l'azote foliaire. Le traitement Mn509 permet le maintien de la teneur foliaire en azote pendant 11 jours après le traitement en retardant la phase de remobilisation de l'azote foliaire.

### EXEMPLE 29 - Efficacité nutritive d'une suspension de carbonate de manganèse selon l'invention sur une culture de soja carencé en manganèse.

**Tableau 4**

| | |
|---|---|
| 7 septembre | Semis de graines de soja sur perlite humidifiée |
| 20 septembre | repiquage des plants de soja sur sable de Valmarque lavé à l'acide chlorhydrique |
| 22 septembre | Apport d'une composition nutritive de Mn à 10 % de la dose prescrite |
| 29 septembre | Apport d'une solution nutritive sans Mn |
| 6 octobre | Renouvellement de la solution nutritive sans Mn |
| 13 octobre | Renouvellement de la solution nutritive sans Mn |
| 20 octobre | Renouvellement de la solution nutritive sans Mn |
| 27 octobre | Renouvellement de la solution nutritive sans Mn |
| 2 novembre | 1^{ère} Récolte pour analyse à J₀ et traitement foliaire |
| 3 novembre | Renouvellement de la solution nutritive sans Mn |
| | 2^{ème} Récolte pour analyse à J₁ |
| 4 novembre | 3^{ème} Récolte pour analyse à J₂ |
| 5 novembre | 4^{ème} Récolte pour analyse à J₃ |
| 8 novembre | 5^{ème} Récolte pour analyse à J₆ |
| 9 novembre | Renouvellement de la solution nutritive sans Mn |
| 11 novembre | 6^{ème} Récolte pour analyse à J₉ |

On choisit des plants de soja (variété Amphor) carencés en manganèse.

Chacune des parcelles choisies reçoit un traitement par nébulisation avec l'une des compositions de traitement T₁, T₂, T₃, T₄, T₅ et T₆ décrites au tableau 5 ci-après. Chaque parcelle de culture de soja carencé en manganèse est traitée avec 100 mL de bouillie (T₁, T₂, T₃, T₄, T₅ et T₆) comprenant 4 g de manganèse (MnSO₄ et/ou MnCO₃) correspondant à une dose de traitement de 500 g/ha de manganèse.

**Tableau 5**

| Composition de traitement | Manganèse appliqué (en g/ha) | Composition de traitement | mL de solution mère dans 100 mL |
|---|---|---|---|
| T₁ | | Eau | 0 |
| T₂ | 400 (MnCO₃) | Exemple 26 | 10 |
| T₃ | 500 (MnCO₃) | Exemple 4 | 8 |
| T₄ | 500 (MnCO₃) | Exemple 6 | 6,9 |
| | 80 (MnSO₄) | | |
| T₅ | 600 (MnCO₃) | Exemple 7 | 6,7 |
| T₆ | 500 (MnCO₃) | Exemple 15 | 8 |

Pour chaque analyse de la teneur foliaire en manganèse à J₀, J₁, J₂, J₃, J₆ et J₉, les feuilles de soja prélevées sont lavées une fois avec une solution à 2 % d'acide acétique dans l'eau déminéralisée, puis deux fois à l'eau déminéralisée. Les feuilles sont ensuite séchées pendant 48 h à la température de 75°C. Trois analyses sont réalisées en parallèle.

Les compositions nutritives T₂, T₃, T₄, T₅ et T₆ permettent une assimilation du manganèse rapide dès l'application du traitement et pendant un à deux jours suivant l'application du traitement. L'application de la composition T₂ (ECG-C₂ et huile de jojoba, MnCO₃ à 400 g/ha) montre une assimilation soutenue et croissante du manganèse qui augmente de 130 ppm à 160 ppm entre le deuxième et le neuvième jour suivants le traitement.

Les compositions T₃, T₄ et T₅ (ECG-C₂) montrent une assimilation initiale (premier et deuxième jours) forte et transitoire, puis un regain d'assimilation aux alentours des septième et huitième jours suivants le traitement.

La composition T₆ (OECG-C₂) montre une assimilation initiale (premier jour) modérée et transitoire, mais un regain d'assimilation qui est maximale à quatre jours et soutenue jusqu'au neuvième jour suivant le traitement.

Le choix des esters carboniques de glycérol et la quantité de solide à l'état divisé appliquée sur les végétaux permettent de moduler le profil d'assimilation du solide à l'état divisé dans les végétaux.

### EXEMPLE 30 - Traitement de semences d'orge.

On traite les semences d'un végétal par exemple choisi dans le groupe formé du maïs, de l'orge, du colza et d'une plante fourragère telle que le Raygrass. Pour ce faire, on choisit des semences d'orge (variété Orjoie, RAGT) présentant un poids de 17,1 g pour 300 graines. On réalise un enrobage/pelliculage de ces semences avec une composition comprenant des oligomères (OECG-C₂) telle que décrite à l'exemple 14 à raison d'une quantité maximale de 1 L de composition d'oligomères par tonne de semence. On mélange les semences et la composition d'oligomère et on laisse le mélange au repos pour séchage.

On analyse la faculté germinative de semences d'orge traitées avec la composition comprenant des oligomères (OECG-C₂) à raison de 0,1, 0,25, 0,5 et 1 L/tonne de semence et on compare la valeur de la faculté germinative obtenue dans chacune de ces conditions avec la valeur de la faculté germinative de semences "témoin" traitées avec une même quantité d'eau. La faculté germinative, exprimée en pourcentage de graines germées au bout de cinq jours, obtenue avec les traitements à 0,1 L/tonne, 0,25 L/tonne, 0,5 L/tonne (90,1 %) et 1L/tonne est supérieure de 3,6 % à 7,3 % à la valeur de la faculté germinative du "témoin" (82,8 %).

### EXEMPLE 31 - Traitement de semences de maïs.

On choisit des semences de maïs (PR33V15, Pioneer) présentant un poids de 60,7 g pour 200 graines. On réalise un enrobage/pelliculage de ces semences avec une composition comprenant des oligomères (OECG-C₂) telle que décrite à l'exemple 14 à raison d'une quantité maximale de 1 L de composition d'oligomères par tonne de semence. On mélange les semences et la composition d'oligomère et on laisse le mélange au repos pour séchage.

On analyse la faculté germinative de semences de maïs traitées avec la composition comprenant des oligomères (OECG-C₂) à raison de 0,1, 0,25 et 0,5 L/tonne de semence et on compare la valeur de la faculté germinative obtenue dans chacune de ces conditions avec la valeur de la faculté germinative de semences "témoin" traitées avec une même quantité d'eau. La faculté germinative, exprimée en pourcentage de graines germées au bout de cinq jours, obtenue avec les traitements à 0,1 L/tonne, 0,25 L/tonne et 0,5 L/tonne (90,1 %) est supérieure de 1,3 % à 1,6 % à la valeur de la faculté germinative du "témoin".

### EXEMPLE 32 - Analyse du développement végétatif des parties aériennes et des parties racinaires de plantes à partir de semences.

On met en culture sur du sable des semences de maïs et d'orge préalablement traitée par enrobage/pelliculage avec une composition comprenant des oligomères (OECG-C₂) telles que décrites aux exemples 30 et 31. Après 15 jours de croissance on sépare et on pèse les parties aériennes (feuilles) et racinaires des plantules. Les traitements avec 0,1 L/tonne et 0,25 L/tonne conduisent à une augmentation respectivement de 29 % et 11 % de la masse totale (parties aériennes et racinaires) des plantules et de 40 % et 23 % de la masse des parties racinaires par rapport aux plantules "témoins".

### EXEMPLE 33 - Effet synergique d'une composition selon l'invention comprenant des oligomères (OECG-C₂) et d'un complément nutritionnel minéral.

On réalise un enrobage/pelliculage de semences de maïs tel que décrit à l'exemple 31 avec une composition comprenant du manganèse (SeedFlow Mn) à raison de 3L/tonne de semences et la composition comprenant des oligomères (OECG-C₂) à raison de 0,25 L/tonne de semences. On observe une augmentation de la faculté germinative des semences traitées par le manganèse et la composition d'oligomères (OECG-C₂) de 3 % par rapport à la faculté germinative de semences traitées avec la composition de manganèse ou avec la composition d'oligomères.

### EXEMPLE 34 - Effet synergique d'une composition selon l'invention comprenant des oligomères (OECG-C₂) et d'un champignon endomycorhizien.

On réalise un enrobage/pelliculage de semences d'orge tel que décrit à l'exemple 30 avec la composition comprenant des oligomères (OECG-C₂) et un champignon endomycorhizien. On observe une augmentation de la faculté germinative des semences traitées par le champignon endomycorhizien par rapport à des semences "témoin" et une augmentation de l'ordre de 2 % de la faculté germinative des semences traitées par le champignon endomycorhizien et par la composition comprenant des oligomères (OECG-C₂) par rapport à la faculté germinative des semences traitées par un champignon endomycorhizien seul.

### EXEMPLE 35 - Stimulation de la photosynthèse.

On applique par pulvérisation sur des plants de soja une composition nutritive (Mn/ OECG-C_{11:1}) comprenant des oligomères (OECG-C_{11:1}) et du carbonate de manganèse telle que décrite aux exemples 22 et 23 et à raison de 500 grammes de carbonate de manganèse par hectare de culture. On réalise, immédiatement après l'application de la composition nutritive, une mesure de la fluorescence chlorophyllienne sur des feuilles de plants de soja ayant reçu la composition nutritive et sur des feuilles de plants de soja n'ayant pas reçu la composition nutritive à titre de contrôle.

On observe, une valeur d'efficacité de la photosynthèse qui est augmentée (0,836) pour le traitement avec la composition nutritive (Mn/ OECG-C_{11:1}) par rapport à la valeur d'efficacité de la photosynthèse (0,831) du contrôle non traité par l'oligomère.

### EXEMPLE 36 - Stimulation de la photosynthèse.

On applique par pulvérisation sur des plants de soja une composition nutritive (Mn/ OECG-C_{18:1}) comprenant des oligomères (OECG-C_{18:1}) et du carbonate de manganèse telle que décrite aux exemples 24 et 25 et à raison de 500 grammes de carbonate de manganèse par hectare de culture. On réalise, immédiatement après l'application de la composition nutritive, une mesure de la fluorescence chlorophyllienne sur des feuilles de plants de soja ayant reçu la composition nutritive et sur des feuilles de plants de soja n'ayant pas reçu la composition nutritive à titre de contrôle.

On observe, une valeur d'efficacité de la photosynthèse qui est augmentée (0,848) pour le traitement avec la composition nutritive (Mn/ OECG-C_{18:1}) par rapport à la valeur d'efficacité de la photosynthèse (0,831) du contrôle non traité par l'oligomère.

## Revendications

1. Procédé de traitement de végétaux comprenant l'application d'au moins un composé, dit ester carbonique de glycérol, comprenant au moins une fonction ester carboxylique formée par :
- un groupement dérivé de l'acide carbonique de formule suivante : 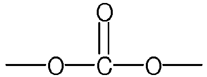 dans laquelle chaque atome d'hydrogène de l'acide carbonique est substitué par un groupement organique distinct de l'hydrogène, et ;
- au moins un groupement dérivé du glycérol de formule suivante : 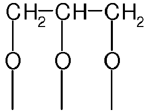 dans laquelle au moins un atome d'hydrogène des groupements hydroxyles du glycérol est substitué par un groupement organique distinct de l'hydrogène, à l'exception du carbonate cyclique de glycérol à cinq chaînons ;
dans lequel au moins un ester carbonique de glycérol est choisi dans le groupe formé :
∘ des esters carboniques linéaires de glycérol présentant au moins un groupement d'atomes de formule (I) générale suivante : 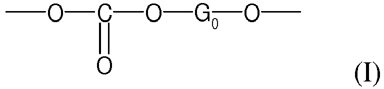 dans laquelle :
- G₀ est choisi dans le groupe formé :
▪ des groupements propylènes α/α'-acylés de formule (II) générale suivante : 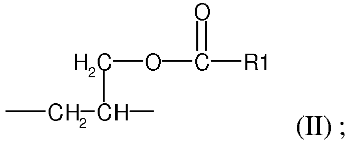
▪ des groupements propylènes β-acylés de formule (III) générale suivante : 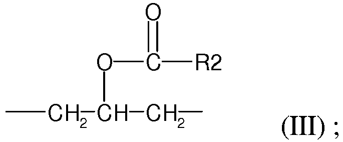
▪ du groupement propylène α/α'-hydroxylé de formule (IV) suivante : 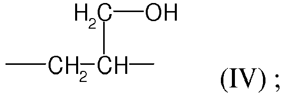
▪ du groupement propylène β-hydroxylé de formule (V) suivante : 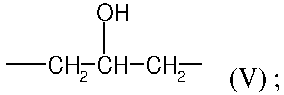 dans lesquelles R1 et R2 sont des groupements organiques formés d'éléments choisis dans le groupe formé du carbone (C), de l'hydrogène (H) et de l'oxygène (O) ;
∘ des esters carboniques cycliques de glycérol α/α'-acylés de formule (VIII) générale suivante : 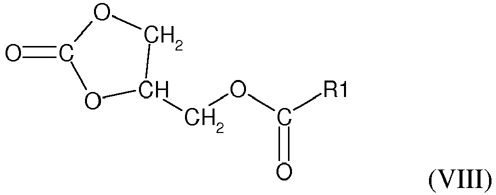 dans laquelle R1 est un groupement organique formé d'éléments choisis dans le groupe formé du carbone (C), de l'hydrogène (H) et de l'oxygène (O), et ;
∘ des esters carboniques cycliques de glycérol β-acylés de formule (IX) générale suivante : 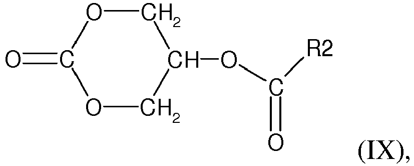 dans laquelle R2 est un groupement organique formé d'éléments choisis dans le groupe formé du carbone (C), de l'hydrogène (H) et de l'oxygène (O).

2. Procédé selon la revendication 1, **caractérisé en ce qu'**au moins un ester carbonique de glycérol est choisi dans le groupe formé des esters carboniques linéaires de glycérol acylés de formule (X) générale suivante : 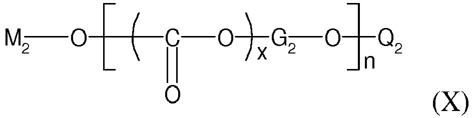 dans laquelle ;
- x est un nombre entier égal à 0 ou à 1 pouvant varier dans la formule (X) selon chaque groupement de formule (X-a) : 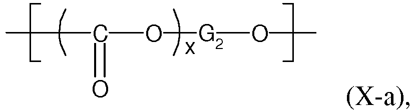 x n'étant pas toujours nul ;
- n est un nombre entier compris entre 1 et 20 -notamment compris entre 1 et 10-, bornes incluses ;
- Q₂ est choisi dans le groupe formé de l'hydrogène (H) et des groupements organiques formés d'au moins deux atomes liés par des liaisons covalentes, lesdits atomes appartenant au groupe formé du carbone (C), de l'hydrogène (H) et de l'oxygène (O), et ;
- M₂ représente un groupement organique formé d'au moins deux atomes liés par des liaisons covalentes, lesdits atomes appartenant au groupe formé du carbone (C), de l'hydrogène (H) et de l'oxygène (O), et ;
- G₂ représente un groupement d'atomes choisi dans le groupe formé :
▪ des groupements propyle α/α'-acylés de formule (II) générale, et ;
▪ des groupements propyle β-acylés de formule (III) générale.

3. Procédé selon la revendication 2, **caractérisé en ce que** l'ester carbonique linéaire de glycérol α/α'-acylé de formule générale (X) présente une masse molaire supérieure à 400 g/mole.

4. Procédé selon l'une des revendications 1 à 3, **caractérisé en ce que** R1 et R2 sont des groupements hydrocarbonés aliphatiques comprenant de 1 à 25 atomes de carbone.

5. Procédé selon l'une des revendications 1 à 4, **caractérisé en ce qu'**on applique au moins un ester carbonique de glycérol en association avec au moins un agent actif autre qu'un ester carbonique de glycérol, choisi dans le groupe formé des agents actifs sur la germination de semences, des agents de contrôle de la croissance, des agents de développement de végétaux, des agents de stimulation de la photosynthèse et des nutriments pour végétaux.

6. Procédé selon l'une des revendications 1 à 5, **caractérisé en ce qu'**on applique au contact de végétaux une composition nutritive comprenant au moins un ester carbonique de glycérol et au moins un solide à l'état divisé comprenant au moins un composé choisi dans le groupe formé des éléments nutritifs des végétaux.

7. Procédé selon l'une des revendications 1 à 6, **caractérisé en ce qu'**on applique au moins un ester carbonique de glycérol au contact de semences de végétaux.

8. Procédé selon l'une des revendications 1 à 7, **caractérisé en ce qu'**on applique au moins un ester carbonique de glycérol au contact de parties aériennes de végétaux.

9. Procédé selon l'une des revendications 1 à 8, **caractérisé en ce qu'**on applique au moins un ester carbonique de glycérol au contact de parties souterraines de végétaux.

10. Composition comprenant :
- au moins un composé, dit ester carbonique de glycérol, comprenant au moins une fonction ester carboxylique formée par :
∘ un groupement dérivé de l'acide carbonique de formule suivante : 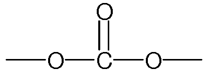 dans laquelle chaque atome d'hydrogène de l'acide carbonique est substitué par un groupement organique distinct de l'hydrogène, et ;
∘ au moins un groupement dérivé du glycérol de formule suivante : 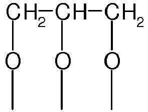 dans laquelle au moins un atome d'hydrogène des groupements hydroxyles du glycérol est substitué par un groupement organique distinct de l'hydrogène, à l'exception du carbonate cyclique de glycérol à cinq chaînons, et
- au moins un agent actif choisi dans le groupe formé des agents actifs sur la germination de semences, des agents de fertilisation et des nutriments pour végétaux ;
dans laquelle au moins un ester carbonique de glycérol est choisi dans le groupe formé :
∘ des esters carboniques linéaires de glycérol présentant au moins un groupement d'atomes de formule (I) générale suivante : 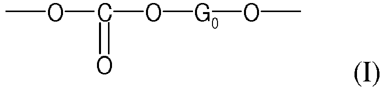 dans laquelle G₀ est choisi dans le groupe formé :
▪ des groupements propylènes α/α'-acylés de formule (II) générale suivante : 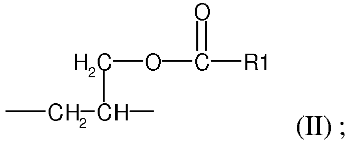
▪ des groupements propylènes β-acylés de formule (III) générale suivante : 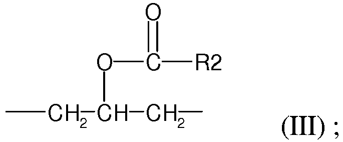
▪ du groupement propylène α/α' -hydroxylé de formule (IV) suivante : 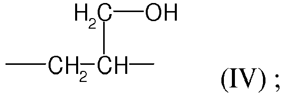
▪ du groupement propylène β-hydroxylé de formule (V) suivante : 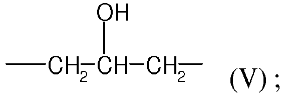
dans lesquelles R1 et R2 sont des groupements organiques formés d'éléments choisis dans le groupe formé du carbone (C), de l'hydrogène (H) et de l'oxygène (O) ;
∘ des esters carboniques cycliques de glycérol α/α'-acylés de formule (VIII) générale suivante : 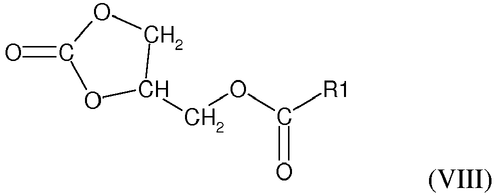 dans laquelle R1 est un groupement organique formé d'éléments choisis dans le groupe formé du carbone (C), de l'hydrogène (H) et de l'oxygène (O), et ;
∘ des esters carboniques cycliques de glycérol β-acylés de formule (IX) générale suivante : 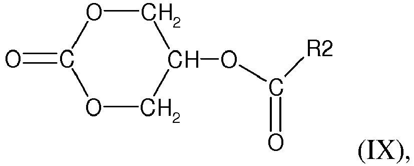 dans laquelle R2 est un groupement organique formé d'éléments choisis dans le groupe formé du carbone (C), de l'hydrogène (H) et de l'oxygène (O).

11. Composition selon la revendication 10, **caractérisée en ce qu'**elle comprend au moins un ester carbonique de glycérol, au moins un solide à l'état divisé comprenant au moins un composé choisi dans le groupe formé des éléments nutritifs des végétaux et un excipient phyto-acceptable autre qu'un ester carbonique de glycérol.

12. Utilisation comme intrant en agriculture d'au moins un composé, dit ester carbonique de glycérol, comprenant au moins une fonction ester carboxylique formée par :
∘ un groupement dérivé de l'acide carbonique de formule suivante : 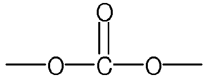 dans laquelle chaque atome d'hydrogène de l'acide carbonique est substitué par un groupement organique distinct de l'hydrogène, et ;
∘ au moins un groupement dérivé du glycérol de formule suivante : 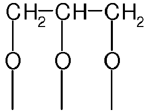 dans laquelle au moins un atome d'hydrogène des groupements hydroxyles du glycérol est substitué par un groupement organique distinct de l'hydrogène, à l'exception du carbonate cyclique de glycérol à cinq chaînons ; dans laquelle au moins un ester carbonique de glycérol est choisi dans le groupe formé :
∘ des esters carboniques linéaires de glycérol présentant au moins un groupement d'atomes de formule (I) générale suivante : 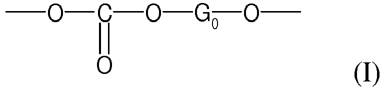 dans laquelle G₀ est choisi dans le groupe formé :
▪ des groupements propylènes α/α'-acylés de formule (II) générale suivante : 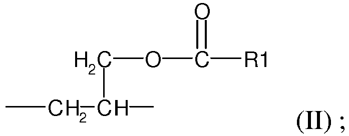
▪ des groupements propylènes β-acylés de formule (III) générale suivante : 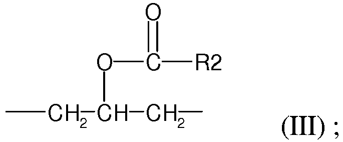
▪ du groupement propylène α/α' -hydroxylé de formule (IV) suivante : 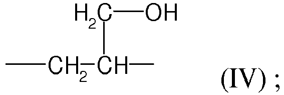
▪ du groupement propylène β-hydroxylé de formule (V) suivante : 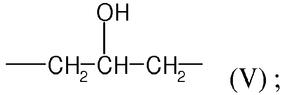 dans lesquelles R1 et R2 sont des groupements organiques formés d'éléments choisis dans le groupe formé du carbone (C), de l'hydrogène (H) et de l'oxygène (O) ;
∘ des esters carboniques cycliques de glycérol α/α' -acylés de formule (VIII) générale suivante : 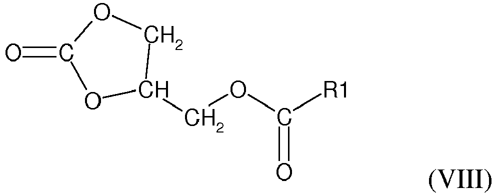 dans laquelle R1 est un groupement organique formé d'éléments choisis dans le groupe formé du carbone (C), de l'hydrogène (H) et de l'oxygène (O), et ;
∘ des esters carboniques cycliques de glycérol β-acylés de formule (IX) générale suivante : 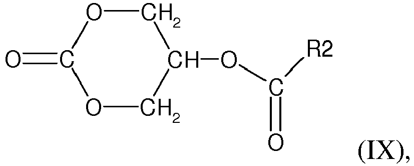 dans laquelle R2 est un groupement organique formé d'éléments choisis dans le groupe formé du carbone (C), de l'hydrogène (H) et de l'oxygène (O).

13. Utilisation selon la revendication 12, **caractérisée en ce qu'**on applique au moins un ester carbonique de glycérol au contact d'un végétal choisi dans le groupe formé des arbres fruitiers, des arbres et arbustes d'ornementation, des plantes potagères, des céréales, des cultures florales et des plantes aromatiques.

## Patentansprüche

1. Verfahren zur Behandlung von Pflanzen, umfassend die Anwendung von mindestens einer Verbindung, bezeichnet als Glycerolcarbonester, umfassend mindestens eine Carboxylesterfunktion, gebildet aus:
- einer Gruppierung, abgeleitet aus der Kohlensäure mit der folgenden Formel: 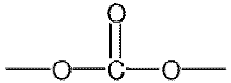 wobei jedes Wasserstoffatom der Kohlensäure durch eine organische Gruppierung substituiert ist, die verschieden von Wasserstoff ist, und;
- mindestens eine Gruppierung, abgeleitet vom Glycerol mit der folgenden Formel: 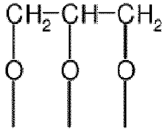 wobei mindestens ein Wasserstoffatom der Hydroxylgruppierungen des Glycerols durch eine organische Gruppierung substituiert ist, die verschieden von Wasserstoff ist, mit Ausnahme des cyclischen Glycerolcarbonats mit fünf Kettengliedern;
wobei mindestens ein Glycerolcarbonester ausgewählt ist aus der Gruppe, gebildet aus:
∘ linearen Glycerolcarbonestern, die mindestens eine Atomgruppierung mit der folgenden allgemeinen Formel (I) aufweisen: 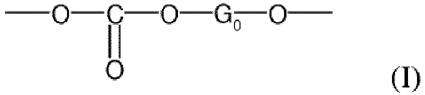 wobei:
- G*₀* ausgewählt ist aus der Gruppe, gebildet aus:
▪ α/α'-acylierten Propylengruppierungen mit der folgenden allgemeinen Formel (II): 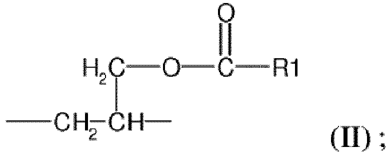
▪ β-acylierten Propylengruppierungen mit der folgenden allgemeinen Formel (III): 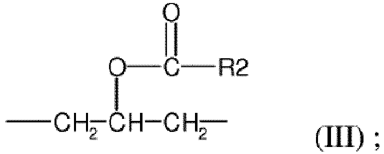
▪ der α/α'-hydroxylierten Propylengruppierung mit der folgenden allgemeinen Formel (IV): 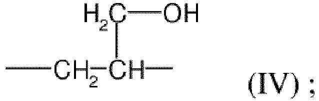
▪ der β-hydroxylierten Propylengruppierung mit der folgenden allgemeinen Formel (V): 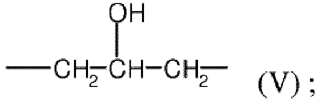 wobei R1 und R2 organische Gruppierungen sind, gebildet aus Elementen, ausgewählt aus der Gruppe, gebildet aus Kohlenstoff (C), Wasserstoff (H) und Sauerstoff (0);
∘ α/α'-acylierten cyclischen Glycerolcarbonestern mit der folgenden allgemeinen Formel (VIII): 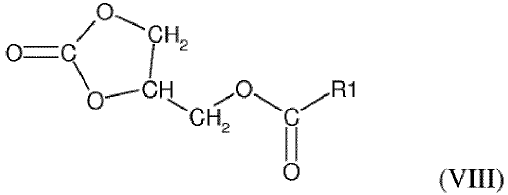 wobei R1 eine organische Gruppierung ist, gebildet aus Elementen, ausgewählt aus der Gruppe, gebildet aus Kohlenstoff (C), Wasserstoff (H) und Sauerstoff (0), und;
∘ β-acylierten cyclischen Glycerolcarbonestern mit der folgenden allgemeinen Formel (IX): 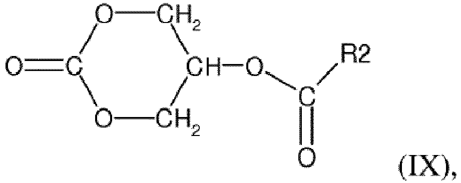 wobei R2 eine organische Gruppierung ist, gebildet aus Elementen, ausgewählt aus der Gruppe, gebildet aus Kohlenstoff (C), Wasserstoff (H) und Sauerstoff (0).

2. Verfahren nach Anspruch 1, **dadurch gekennzeichnet, dass** mindestens ein Glycerolcarbonester ausgewählt ist aus der Gruppe, gebildet aus acylierten linearen Glycerolcarbonestern mit der folgenden allgemeinen Formel (X): 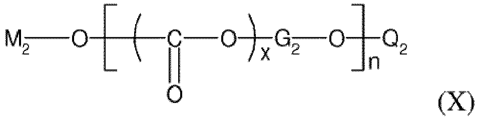 Wobei;
- x eine ganze Zahl gleich 0 oder 1 ist, die in der Formel (X) gemäß jeder Gruppierung mit der Formel (X-a) variieren kann: 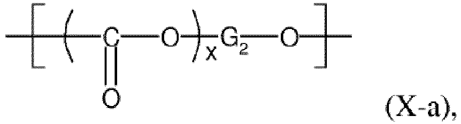 wobei x nicht immer null ist;
- n eine ganze Zahl zwischen 1 und 20 ist,- insbesondere zwischen 1 und 10 - darin eingeschlossen die Schranken;
- Q₂ ausgewählt ist aus der Gruppe, gebildet aus Wasserstoff (H) und organischen Gruppierungen, gebildet aus mindestens zwei Atomen, die durch kovalente Bindungen verbunden sind, wobei die Atome Teil der Gruppe sind, gebildet aus Kohlenstoff (C), Wasserstoff (H) und Sauerstoff (0), und;
- M₂ eine organische Gruppierung darstellt, gebildet aus mindestens zwei Atomen, die durch kovalente Bindungen verbunden sind, wobei die Atome Teil der Gruppe sind, gebildet aus Kohlenstoff (C), Wasserstoff (H) und Sauerstoff (0), und;
- G₂ eine Gruppierung von Atomen darstellt, ausgewählt aus der Gruppe, gebildet aus:
▪ α/α'-acylierten Propylgruppierungen mit der folgenden allgemeinen Formel (II), und;
▪ β-acylierten Gruppierungen mit der allgemeinen Formel (III).

3. Verfahren nach Anspruch 2, **dadurch gekennzeichnet, dass** das α/α'-acylierte lineare Glycerolcarbonester mit der allgemeinen Formel (X) eine Molmasse von mehr als 400 g/mol aufweist.

4. Verfahren nach einem der Ansprüche 1 bis 3, **dadurch gekennzeichnet, dass** R1 und R2 aliphatische Kohlenwasserstoffgruppierungen sind, umfassend 1 bis 25 Kohlenstoffatome.

5. Verfahren nach einem der Ansprüche 1 bis 4, **dadurch gekennzeichnet, dass** mindestens ein Glycerolcarbonester in Verbindung mit mindestens einem Wirkstoff, mit Ausnahme eines Glycerolcarbonesters, angewendet wird, ausgewählt aus der Gruppe, gebildet aus Wirkstoffen für die Keimung von Saatgut, Mitteln zur Steuerung des Wachstums, Mitteln zur Entwicklung von Pflanzen, Mitteln zur Stimulierung der Photosynthese und Nährstoffen für Pflanzen.

6. Verfahren nach einem der Ansprüche 1 bis 5, **dadurch gekennzeichnet, dass** in Kontakt mit Pflanzen eine Nährstoffzusammensetzung angewendet wird, umfassend mindestens einen Glycerolcarbonester und mindestens einen Feststoff im geteilten Zustand, umfassend mindestens eine Verbindung, ausgewählt aus der Gruppe, gebildet aus Nährstoffelementen der Pflanzen.

7. Verfahren nach einem der Ansprüche 1 bis 6, **dadurch gekennzeichnet, dass** mindestens ein Glycerolcarbonester in Kontakt mit Saatgut von Pflanzen angewendet wird.

8. Verfahren nach einem der Ansprüche 1 bis 7, **dadurch gekennzeichnet, dass** mindestens ein Glycerolcarbonester in Kontakt mit oberirdischen Teilen von Pflanzen angewendet wird.

9. Verfahren nach einem der Ansprüche 1 bis 8, **dadurch gekennzeichnet, dass** mindestens ein Glycerolcarbonester in Kontakt mit unterirdischen Teilen von Pflanzen angewendet wird.

10. Zusammensetzung, umfassend:
- mindestens eine Verbindung, bezeichnet als Glycerolcarbonester, umfassend mindestens eine Carboxylesterfunktion, gebildet aus:
∘ einer Gruppierung, abgeleitet aus der Kohlensäure mit der folgenden Formel: 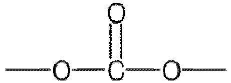 wobei jedes Wasserstoffatom der Kohlensäure durch eine organische Gruppierung substituiert ist, die verschieden von Wasserstoff ist, und;
∘ mindestens eine Gruppierung, abgeleitet aus dem Glycerol mit der folgenden Formel: 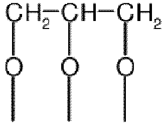 wobei mindestens ein Wasserstoffatom der Hydroxylgruppierungen des Glycerols durch eine organische Gruppierung substituiert ist, die verschieden von Wasserstoff ist, mit Ausnahme des cyclischen Glycerolcarbonats mit fünf Kettengliedern, und
- mindestens einen Wirkstoff, ausgewählt aus der Gruppe, gebildet aus Wirkstoffen für die Keimung von Saatgut, Düngemitteln und Nährstoffen für Pflanzen;
wobei mindestens ein Glycerolcarbonester ausgewählt ist aus der Gruppe, gebildet aus:
∘ linearen Glycerolcarbonestern, die mindestens eine Atomgruppierung mit der folgenden allgemeinen Formel (I) aufweisen: 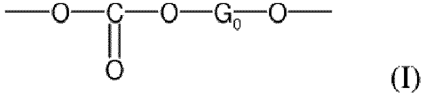 wobei G*₀* ausgewählt ist aus der Gruppe, gebildet aus:
▪ α/α'-acylierten Propylengruppierungen mit der folgenden allgemeinen Formel (II): 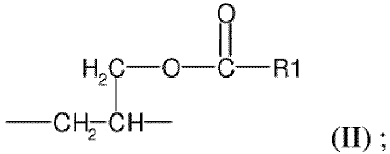
▪ β-acylierten Propylengruppierungen mit der folgenden allgemeinen Formel (III): 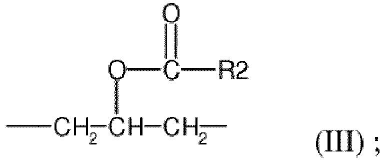
▪ der α/α'-hydroxylierten Propylengruppierung mit der folgenden allgemeinen Formel (IV): 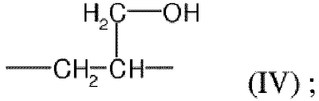
▪ der β-hydroxylierten Propylengruppierung mit der folgenden allgemeinen Formel (V): 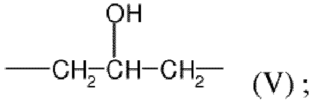 wobei R1 und R2 organische Gruppierungen sind, gebildet aus Elementen, ausgewählt aus der Gruppe, gebildet aus Kohlenstoff (C), Wasserstoff (H) und Sauerstoff (0);
∘ α/α'-acylierten cyclischen Glycerolcarbonestern mit der folgenden allgemeinen Formel (VIII): 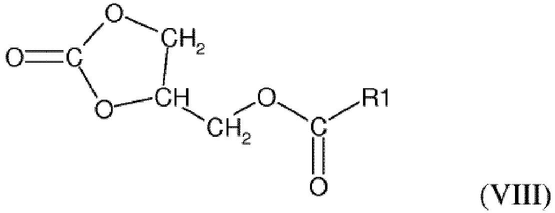 wobei R1 eine organische Gruppierung ist, gebildet aus Elementen, ausgewählt aus der Gruppe, gebildet aus:Kohlenstoff (C), Wasserstoff (H) und Sauerstoff (0), und;
∘ β-acylierten cyclischen Glycerolcarbonestern mit der folgenden allgemeinen Formel (IX): 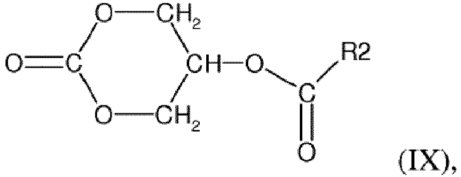 wobei R2 eine organische Gruppierung ist, gebildet aus Elementen, ausgewählt aus der Gruppe, gebildet aus Kohlenstoff (C), Wasserstoff (H) und Sauerstoff (0).

11. Zusammensetzung nach Anspruch 10, **dadurch gekennzeichnet, dass** sie mindestens ein Glycerolcarbonester umfasst, mindestens einen Feststoff im geteilten Zustand, umfassend mindestens eine Verbindung, ausgewählt aus der Gruppe, gebildet aus Nährstoffelementen der Pflanzen und einem Phyto-annehmbaren Trägerstoff, mit Ausnahme eines Glycerolcarbonesters.

12. Verwendung als Einsatzmittel in der Landwirtschaft von mindestens einer Verbindung, bezeichnet als Glycerolcarbonester umfassend mindestens eine Carboxylesterfunktion, gebildet aus:
∘ einer Gruppierung, abgeleitet aus Kohlensäure mit der folgenden Formel: 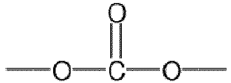 wobei jedes Wasserstoffatom der Kohlensäure durch eine organische Gruppierung substituiert ist, die verschieden von Wasserstoff ist, und;
∘ mindestens eine Gruppierung, abgeleitet aus Glycerol mit der folgenden Formel: 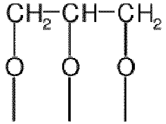 wobei mindestens ein Wasserstoffatom der Hydroxylgruppierungen des Glycerols durch eine organische Gruppierung substituiert ist, die verschieden von Wasserstoff ist, mit Ausnahme des cyclischen Glycerolcarbonats mit fünf Kettengliedern;
wobei mindestens ein Glycerolcarbonester ausgewählt ist aus der Gruppe, gebildet aus:
∘ linearen Glycerolcarbonestern, die mindestens eine Atomgruppierung mit der folgenden allgemeinen Formel (I) aufweisen: 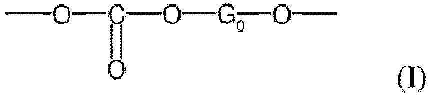 wobei G*₀* ausgewählt ist aus der Gruppe, gebildet aus:
▪ α/α'-acylierten Propylengruppierungen mit der folgenden allgemeinen Formel (II): 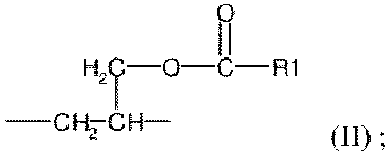
▪ β-acylierten Propylengruppierungen mit der folgenden allgemeinen Formel (III): 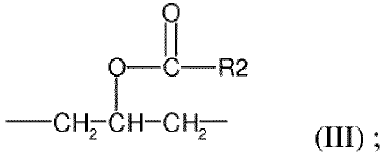
▪ der α/α'-hydroxylierten Propylengruppierung mit der folgenden allgemeinen Formel (IV): 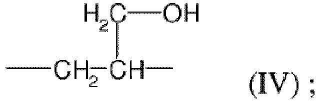
▪ der β-hydroxylierten Propylengruppierung mit der folgenden allgemeinen Formel (V): 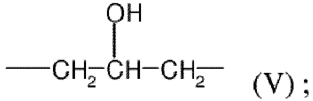 wobei R1 und R2 organische Gruppierungen sind, gebildet aus Elementen, ausgewählt aus der Gruppe, gebildet aus Kohlenstoff (C), Wasserstoff (H) und Sauerstoff (0);
∘ α/α'-acylierten cyclischen Glycerolcarbonestern mit der folgenden allgemeinen Formel (VIII): 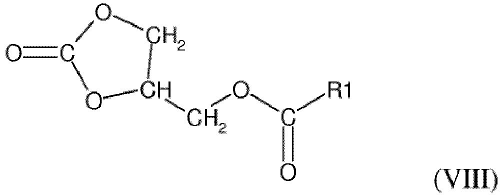 wobei R1 eine organische Gruppierung ist, gebildet aus Elementen, ausgewählt aus der Gruppe, gebildet aus Kohlenstoff (C), Wasserstoff (H) und Sauerstoff (0), und;
∘ β-acylierten cyclischen Glycerolcarbonestern mit der folgenden allgemeinen Formel (IX): 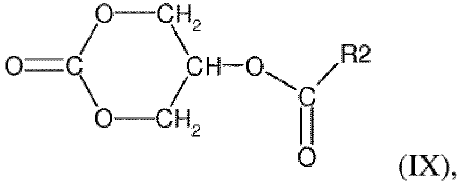 wobei R2 eine organische Gruppierung ist, gebildet aus Elementen, ausgewählt aus der Gruppe, gebildet aus Kohlenstoff (C), Wasserstoff (H) und Sauerstoff (0).

13. Verwendung nach Anspruch 12, **dadurch gekennzeichnet, dass** mindestens ein Glycerolcarbonester in Kontakt mit einer Pflanze angewendet wird, ausgewählt aus der Gruppe, gebildet aus Obstbäumen, Zierbäumen und -sträuchern, Gemüsepflanzen, Getreide, Zierpflanzen und aromatischen Pflanzen.

## Claims

1. Plant treatment method including the application of at least one compound called carbonic ester of glycerol, including at least one carboxylic ester function composed of:
- a group derived from carbonic acid with the following formula: 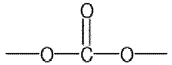 wherein each hydrogen atom in the carbonic acid is substituted by an organic group distinct from hydrogen, and:
- at least one group derived from glycerol with the following formula: 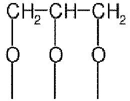 wherein at least one hydrogen atom in the hydroxyl groups of glycerol is substituted by an organic group distinct from hydrogen,
except for cyclic glycerol carbonate with five chains;
wherein at least one carbonic ester of glycerol is chosen from the group formed from:
∘ linear carbonic esters of glycerol with at least one group of atoms with the following general formula (I) : 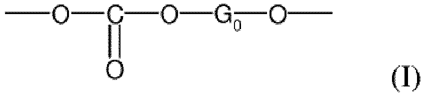 wherein:
- G₀ is chosen from the group composed of:
▪ α/α'-acylated propylene groups with the following general formula (II): 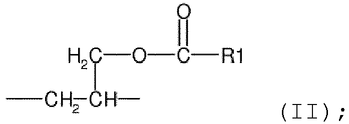
▪ β-acylated propylene groups with the following general formula (III): 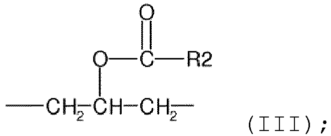
▪ the α/α'-hydroxyl propylene group with the following general formula (IV): 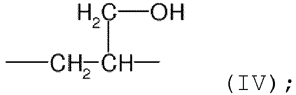
▪ the β-hydroxyl propylene group with the following general formula (V): 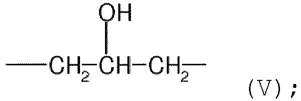 wherein R1 and R2 are organic groups formed from elements chosen from the group composed of carbon (C), hydrogen (H) and oxygen (0);
∘ cyclic α/α'-acylated carbonic esters of glycerol with the following general formula (VIII): 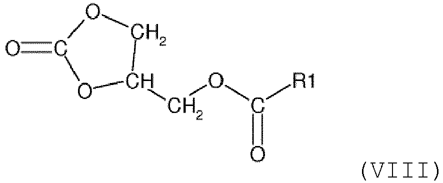 wherein R1 is an organic group formed from elements chosen from the group composed of carbon (C), hydrogen (H) and oxygen (0), and;
∘ cyclic β'-acylated carbonic esters of glycerol with the following general formula (IX): 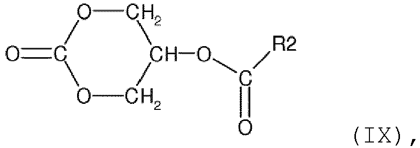 wherein R2 is an organic group formed from elements chosen from the group composed of carbon (C), hydrogen (H) and oxygen (0).

2. Method according to claim 1, **characterised in that** at least one carbonic ester of glycerol is chosen from the group composed of linear acylated carbonic esters of glycerol with the following general formula (X) : 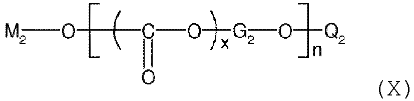 Wherein;
- x is an integer number equal to 0 or 1 that can vary in formula (X) for each group with formula (X-a): 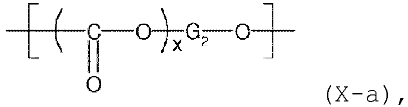 x not always being zero;
- n is an integer number between 1 and 20, and particularly between 1 and 10, including limits;
- Q₂ is chosen from the group composed of hydrogen (H) and organic groups formed from at least two atoms bonded by covalent bonds, said atoms belonging to the group composed of carbon (C), hydrogen (H) and oxygen (0), and;
- M₂ represents an organic group composed of at least two atoms bonded by covalent bonds, said atoms belonging to the group composed of carbon (C), hydrogen (H) and oxygen (0), and;
- G₂ represents a group of atoms chosen from the group composed of:
▪ α/α'-acylated propyl groups with the general formula (II),
and;
▪ β-acylated propyl groups with the general formula (III).

3. Method according to claim 2, **characterised in that** the linear α/α'-acylated carbonic ester of glycerol with general formula (X) has a molar mass of more than 400 g/mole.

4. Method according to one of claims 1 to 3, **characterised in that** R1 and R2 are aliphatic hydrocarbon groups including 1 to 25 carbon atoms.

5. Method according to one of claims 1 to 4, **characterised in that** at least one carbonic acid of glycerol is applied in association with at least one active agent other than a carbonic ester of glycerol, chosen from the group composed of agents active on the germination of seeds, growth control agents, plant development agents, photosynthesis stimulation agents and plant nutrients.

6. Method according to one of claims 1 to 5, **characterised in that** a nutrient composition comprising at least one carbonic ester of glycerol and at least one solid in the divided state comprising at least one compound chosen from the group composed of plant nutrient elements, is applied in contact with plants.

7. Method according to one of claims 1 to 6, **characterised in that** at least one carbonic ester of glycerol is applied in contact with plant seeds.

8. Method according to one of claims 1 to 7, **characterised in that** at least one carbonic ester of glycerol is applied in contact with parts of the plant above the ground.

9. Method according to one of claims 1 to 8, **characterised in that** at least one carbonic ester of glycerol is applied in contact with parts of the plant below the ground.

10. Composition comprising:
- at least one compound, called a carbonic ester of glycerol, comprising at least one carboxylic ester function formed by:
∘ a group derived from carbonic acid with the following formula: 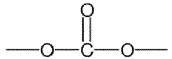 wherein each hydrogen atom in the carbonic acid is substituted by an organic group distinct from hydrogen, and;
∘ at least one group derived from glycerol with the following formula: 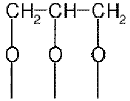 wherein at least one hydrogen atom in the hydroxyl groups of glycerol is substituted by an organic group distinct from hydrogen,
except for cyclic glycerol carbonate with five chains, and
- at least one active agent chosen from the group formed from active agents on germination of seeds, fertilisation agents and nutrients for plants;
wherein at least one carbonic ester of glycerol is chosen from the group formed from:
∘ linear carbonic esters of glycerol with at least one group of atoms with the following general formula (I) : 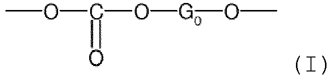 wherein G₀ is chosen among the group composed of:
▪ α/α'-acylated propylene groups with the following general formula (II): 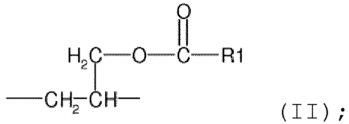
▪ β-acylated propylene groups with the following general formula (III): 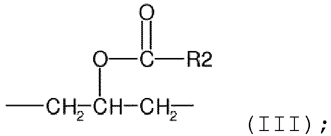
▪ the α/α'-hydroxyl propylene group with the following general formula (IV): 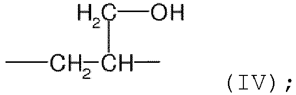
▪ the β-hydroxyl propylene group with the following general formula (V): 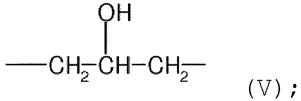 wherein R1 and R2 are organic groups formed from elements chosen from the group composed of carbon (C), hydrogen (H) and oxygen (0);
∘ cyclic α/α'-acylated carbonic esters of glycerol with the following general formula (VIII): 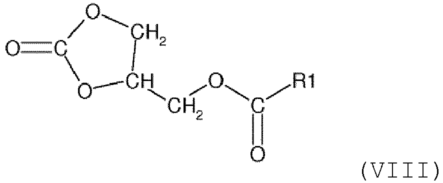 wherein R1 is an organic group formed from elements chosen from the group composed of carbon (C), hydrogen (H) and oxygen (0), and;
∘ cyclic β'-acylated carbonic esters of glycerol with the following general formula (IX): 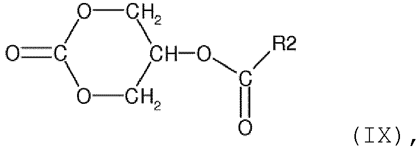 wherein R2 is an organic group formed from elements chosen from the group composed of carbon (C), hydrogen (H) and oxygen (0).

11. Composition according to claim 10, **characterised in that** it comprises at least one carbonic ester of glycerol, at least one solid in the divided state comprising at least one compound chosen from the group composed of plant nutrient elements and a phyto-acceptable excipient other than a carbonic ester of glycerol.

12. Use as an input in agriculture of at least one compound called a carbonic ester of glycerol, comprising at least one carboxylic ester function composed of:
∘ a group derived from carbonic acid with the following formula: 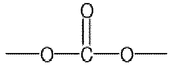 wherein each hydrogen atom in the carbonic acid is substituted by an organic group distinct from hydrogen, and;
∘ at least one group derived from glycerol with the following formula: 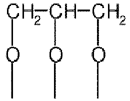 wherein at least one hydrogen atom in the hydroxyl groups of glycerol is substituted by an organic group distinct from hydrogen,
except for cyclic glycerol carbonate with five chains;
wherein at least one carbonic ester of glycerol is chosen among the group composed of:
∘ linear carbonic esters of glycerol with at least one group of atoms with the following general formula (I) : 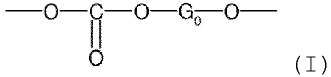 wherein G₀ is chosen among the group composed of:
▪ α/α'-acylated propylene groups with the following general formula (II): 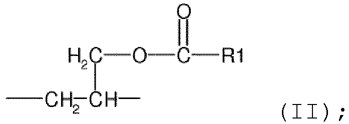
▪ β-acylated propylene groups with the following general formula (III): 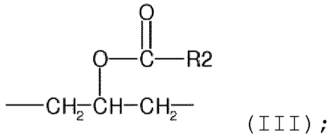
▪ the α/α'-hydroxyl propylene group with the following general formula (IV): 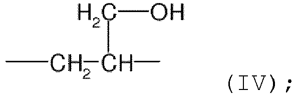
▪ the β-hydroxyl propylene group with the following general formula (V): 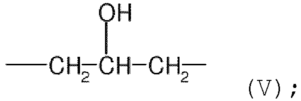 wherein R1 and R2 are organic groups formed from elements chosen from the group composed of carbon (C), hydrogen (H) and oxygen (0);
∘ cyclic α/α'-acylated carbonic esters of glycerol with the following general formula (VIII): 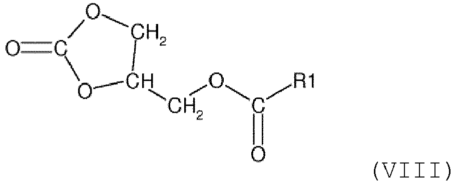 wherein R1 is an organic group formed from elements chosen from the group composed of carbon (C), hydrogen (H) and oxygen (0), and;
∘ cyclic β'-acylated carbonic esters of glycerol with the following general formula (IX): 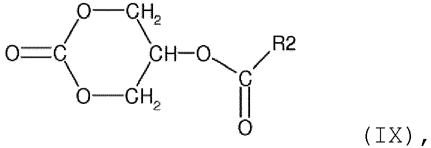 wherein R2 is an organic group formed from elements chosen from the group composed of carbon (C), hydrogen (H) and oxygen (0).

13. Use according to claim 12, **characterised in that** at least one carbonic ester of glycerol is applied in contact with a plant chosen from the group composed of fruit trees, ornamental trees and shrubs, vegetables, cereals, flower crops and aromatic plants.
